# EUROPEAN PATENT APPLICATION

(11) **EP 4 458 412 A1**
(43) Date of publication of application: **06.11.2024**
(21) Application number: 23171966.7
(22) Date of filing: 05.05.2023
(51) Int. Cl.: A61P 19/00, A61P 35/00, A61P 37/00, C07F 9/44

(54) **NOVEL PHOSPHOANTIGEN PRODRUG COMPOUND AND USE THEREOF**

(71) Applicant: University College Cardiff Consultants, Ltd., South Glamorgan Cardiff CF24 0DE (GB); University of Birmingham, Birmingham, West Midlands B15 2TT (GB)
(72) Inventor: MEHELLOU, Youcef, Cardiff, CF10 3NB (GB); WILLCOX, Benjamin, Birmingham, B15 2TT (GB)
(74) Representative: Uexküll & Stolberg

(57) **Abstract**

The invention relates to a novel phosphoantigen (PAg) prodrug compound which provides for a potent activation of therapeutically active γδ T cells. The PAg prodrug compound of the invention can be used for the preparation of therapeutically active γδ T cells as well as in the immunotherapy of different diseases, and in particular different types of cancer. The invention further relates to a method for expanding γδ T-cells *ex vivo* which makes use of the PAg prodrug compound of the invention. Also provided are γδ T-cells which have been expanded with the PAg prodrug compound of the invention and pharmaceutical compositions comprising such expanded cells and/or the PAg prodrug compound of the invention.

## Description

The invention relates to a novel phosphoantigen (PAg) prodrug compound which provides for a potent activation of therapeutically active γδ T cells. The PAg prodrug compound of the invention can be used for the preparation of therapeutically active γδ T cells as well as in the immunotherapy of different diseases, and in particular different types of cancer. The invention further relates to a method for expanding γδ T-cells *ex vivo* which makes use of the PAg prodrug compound of the invention. Also provided are γδ T-cells which have been expanded with the PAg prodrug compound of the invention and pharmaceutical compositions comprising such expanded cells and/or the PAg prodrug compound of the invention.

### BACKGROUND OF THE INVENTION

Present since birth and the dominant subtype of human γδ T-cells in adult peripheral blood, Yγ9/Vδ2 T cells are now established as a key subset of yδ T-cells that is involved in the fight against many diseases such as tuberculosis, leprosy, typhoid, malaria, and toxoplasmosis, see, for example, Morita, C. T.; Jin, C.; Sarikonda, G.; Wang, H. Nonpeptide antigens, presentation mechanisms, and immunological memory of human Vgamma2Vdelta2 T cells: discriminating friend from foe through the recognition of prenyl pyrophosphate antigens. Immunol Rev 2007, 215, 59-76 (hereinafter "Morita et al. 2007").

Studies in primate models have also implicated Yγ9/Vδ2 T cells in immunity to *Mycobacterium tuberculosis,* see Shen, Y; Zhou, D.; Qiu, L.; Lai, X.; Simon, M.; Shen, L.; Kou, Z.; Wang, Q.; Jiang, L.; Estep, J.; Hunt, R.; Clagett, M.; Sehgal, P. K.; Li, Y; Zeng, X.; Morita, C. T.; Brenner, M. B.; Letvin, N. L.; Chen, Z. W. Adaptive immune response of Vgamma2Vdelta2+ T cells during mycobacterial infections. Science (New York, N.Y.) 2002, 295, 2255-8.

These cells have also shown an ability to target and lyse a diverse range of cancer cells *in vitro* spontaneously or upon activation with small molecule activators, see Morita et al, Immunol Rev 2007, 215, 59-76. Together, these observations have made the Yγ9/Vδ2 subset a major focus in the therapeutic exploitation of γδ T-cells, in particular in autologous or allogeneic immunotherapy, see Fisher, J. P.; Heuijerjans, J.; Yan, M.; Gustafsson, K.; Anderson, J. gammadelta T cells for cancer immunotherapy: A systematic review of clinical trials. Oncoimmunology 2014, 3, e27572 (hereinafter "Fisher et al. 2014"). The activation οf γδ T-cells with small molecule activators can be conducted *in vivo* or *ex vivo.*

The treatment of patients with γδ T-cells commonly involves obtaining T cells from donor blood. For example, the T cells can be obtained from peripheral blood mononuclear cells (PBMCs) which are isolated from peripheral blood. The T cells can also be obtained from cord blood mononuclear cells (CBMC) which are isolated from umbilical blood. Another source for suitable T cells are non-haematopoietic tissues. After their isolation, the T cells are cultivated to increase their number which is referred to expansion. To enhance cell growth and/or functionality of the T cells to be expanded, it is known to culture the T cells in the presence of small molecule activators. For example, *ex vivo* expansion of PBMCs in the presence of small molecule activators such as phosphoantigens or aminobisphosphonates provides for γδ T-cells of the Vy9/V62 phenotype; see for example WO 2016/166544 or WO 2018/055191.

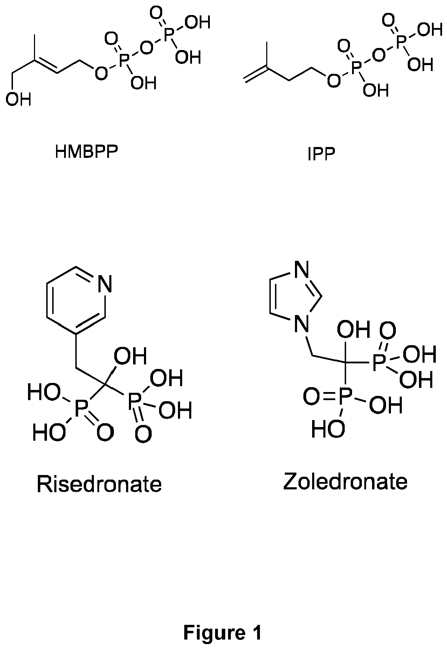

To date, a number of small molecule activators of Yγ9/Vδ2 T cells have been reported. Among these are the naturally-occurring PAgs (*E*)-4-hydroxy-3-methylbut-2-enyl pyrophosphate (HMBPP) and isopentenyl pyrophosphate (IPP), and the two synthetic molecules, risedronate and zoledronate (Figure 1), which are currently used in the clinic to treat osteoporosis and some types of cancer, see Maraka, S.; Kennel, K. A. Bisphosphonates for the prevention and treatment of osteoporosis. BMJ (Clinical research ed.) 2015, 351, h3783; and Roelofs, A. J.; Jauhiainen, M.; Monkkonen, H.; Rogers, M. J.; Monkkonen, J.; Thompson, K. Peripheral blood monocytes are responsible for gammadelta T cell activation induced by zoledronic acid through accumulation of IPPIDMAPP. British journal of haematology 2009, 144, 245-50; and Davey, M. S.; Malde, R.; Mykura, R. C.; Baker, A. T.; Taher, T. E.; Le Duff, C. S.; Willcox, B. E.; Mehellou, Y. Synthesis and Biological Evaluation of ( E)-4-Hydroxy-3-methylbut-2-enyl Phosphate (HMBP) Aryloxy Triester Phosphoramidate Prodrugs as Activators of Vgamma9/Vdelta2 T-Cell Immune Responses. J Med Chem 2018, 61, 2111-2117 (hereinafter "Mehellou et al. 2018").

The most potent Yγ9/Vδ2 T cells activator reported to date is HMBPP, EC₅₀ = 0.00051 µM, which activates T cells by binding the type-1 transmembrane protein butyrophilin 3A1. Although the binding site for these PAgs remained unclear amid conflicting reports as to whether they bind the extracellular or intracellular domains of this transmembrane protein, there is now compelling evidence that supports the notion that HMBPP binds the intracellular B30.2 domain of butyrophilin 3A1.

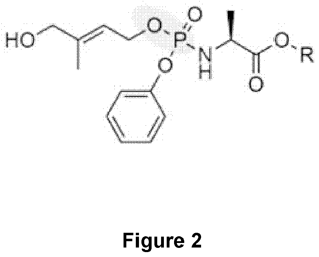

Encouraged by the potency of the HMBPP in activating Vy9/V62 T-cells, aryloxy triester phosphoramidate prodrug technology was recently applied to the monophosphate derivative of HMBPP, *i.e.* HMBP, as a means to improve its drug-like properties, see Mehellou et al. 2018. In this prodrug approach, the monophosphate group is masked by an aryl group and/or an amino acid ester (Figure 2), which are both enzymatically cleaved off inside cells to release the monophosphate or monophosphonate species. As such compounds are prodrugs of PAgs, they are termed ProPAgens to distinguish them from ProTides, which are prodrugs of nucleotides.

Whilst these HMBP ProPAgens exhibited potent activation of Vy9/Vδ2 T-cells (EC₅₀ = 0.45-11 nM), their serum stability disadvantageously was rather low (t_{1/2} < 30 min), primarily due to the cleavage of the -P-O- bond of these active compounds, *ibid.*

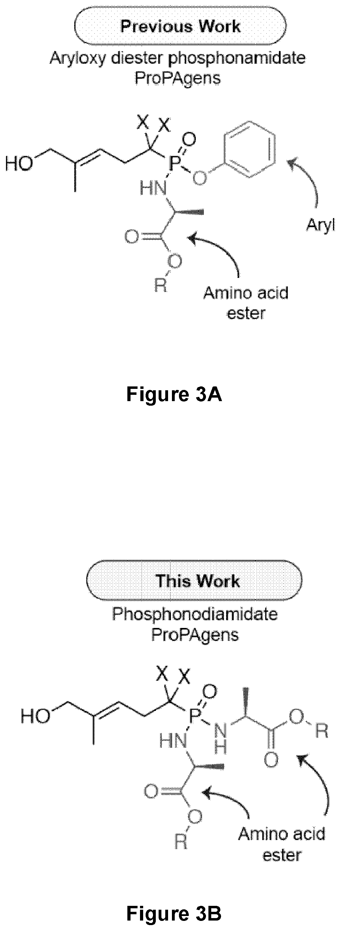

Against this background, aryloxy diester phosphonamidate ProPAgens were designed (Figure 3A), which provide for serum stability (t_{1/2} > 12 h in human serum) and potent activation of Vy9/Vδ2 T-cells (EC₅₀ ranging from 5 fm to 73 nM), see Kadri, H.; Taher, T. E.; Xu, Q.; Sharif, M.; Ashby, E.; Bryan, R. T.; Willcox, B. E.; Mehellou, Y. Aryloxy Diester Phosphonamidate Prodrugs of Phosphoantigens (ProPAgens) as Potent Activators of Vy9/V62 T-Cell Immune Responses. J. Med. Chem. 2020, 63, 11258-11270. (hereinafter "Mehellou et al. 2020). The aryloxy diester phosphonamidate ProPAgens are also disclosed in WO 2020/008189.

However, these aryloxy diester phosphonamidate ProPAgens of Mehellou et al. 2020 can potentially be associated with *in vivo* toxicity due to the aryl ester group at the phosphorous atom. Also, the phosphorous atom in these aryloxy diester phosphonamidate ProPAgens is chiral, giving rise to diasteromeric mixtures. Disadvantageously, different diastereomers may be associated with different biological profiles in terms of pharmacology, toxicology, phar-macokinetics and metabolism, in which case the diasteromeric mixtures need to be separated. Isolation of stereoisomers often is tedious and in general inefficient, cost-extensive, time consuming and difficult to scale up.

Therefore, it is an object of the present invention to provide ProPAgens that retain potent activation of Vy9/Vδ2 T-cells whilst overcoming the challenges associated with stereochemistry and *in vivo* toxicity of previously reported HMBP ProPAgens active compounds.

### SUMMARY OF THE INVENTION

The present invention relates to novel symmetrical amino acid ester-derived phosphonodiamidate ProPAgens according to the claims set out hereinbelow, and pharmaceutical compositions comprising ProPAgens. The present invention also relates to the synthesis of the symmetrical amino acid ester-derived methyl and difluoromethyl phosphonodiamidate ProPAgens.

The present invention involves the application of symmetric amino acid ester groups to phosphoantigens. The resulting products exert advantageous characteristics in terms of stability, metabolism and pharmacological activity.

The ProPAgens of the present invention represent a new class of small molecule activators of Vy9/V62 T cells with potentially improved *in vivo* safety and efficacy. The phosphonodiamidate ProPAgens of the present invention are metabolised to release alcohol and natural amino acids, which are relatively non-toxic compared to phenols released from aryloxy ProPAgens. Also, the ProPAgens of the invention provide for improved water solubility due the use of relatively hydrophilic amino acid ester groups as phosphate masking groups instead of the lipophilic aromatic ring (aryloxy group). Further, due to their symmetrical molecular structure, the number of stereocenters is reduced, which advantageously simplifies synthesis and separation of the target compounds. This comes along with the beneficial effect that undesired product mixtures of stereoisomers having potentially different biological profiles are avoided, thereby increasing reliability of the target compounds when used in the treatment of a subject in need thereof.

The ProPAgens of the present invention exhibit excellent stability in human serum and elicited potent activation of Vy9/V62 T cells, which was translated into potent *in vitro* killing of the urinary bladder carcinoma cell line T24. Surprisingly, the ProPAgens of the present invention provide for superior activation of Vy9/V62 T cells compared to prior art compounds such as zoledronate and HMBPP.

The combination of high, specificity, serum stability and potency profiles of these new phosphonate ProPAgens makes them suitable for development as new immunotherapeutics for treating a variety of conditions, including proliferative diseases, such as cancer, osteoporosis, various infectious diseases, such as tuberculosis, leprosy, typhoid, malaria, and toxoplasmosis, and/or inflammatory diseases. The phosphonodiamidate ProPAgens of the present invention can be used in a monotherapy regimen and also as part of or subsequent to a clinical regimen to expand γδ T cells *in vivo* or, alternatively, they could be administered to patients receiving adoptive cell therapy with *ex* vivo-expanded γδ T cells, to directly augment Yγ9/Vδ2 T cell-mediated activity, in particular antitumor activity.

The invention further relates to a method for expanding γδ T-cells ex vivo wherein γδ T-cells are cultured in the presence of a ProPAgens according to the invention or a pharmaceutical composition comprising such ProPAgens. Also provided is a population of expanded γδ T cells or pharmaceutical compositions comprising such expanded γδ T cells.

### DETAILED DESCRIPTION OF THE INVENTION

According to a first aspect of the invention, there is provided a compound according to General Formula (I) including all tautomers thereof: 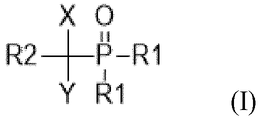 wherein R1 represents an amino acid ester radical according to General Formula (II): 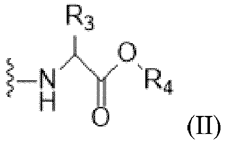 wherein R3 represents H, or a saturated or unsaturated and optionally substituted hydrocarbon chain; R4 represents a saturated or unsaturated and optionally substituted hydrocarbon chain; and wherein both R1 are identical; and
R2 represents an optionally substituted C₂₋₂₀ alkyl, C₄₋₂₀ alkenyl or C₂₋₂₀ alcohol radical; and
X and Y each independently represent H or halo;
or a salt thereof,
and wherein R1 is an amino acid ester radical according to General Formula (II) derived from L-alanine, L-leucine, L-isoleucine or L-methionine, most preferably L-alanine,
and wherein R2 is a radical according to Formula (III) or Formula (IV), wherein R5 is selected from OH, OR6, SH, SR6, NH₂ or NHR6 and is preferably OH, and wherein R6 represents C₁₋₄ alkyl:

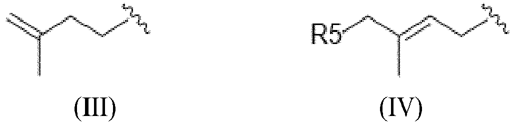

These R2 substituents are analogous to those found in the naturally-occurring PAgs IPP (Formula (III)), and HMBPP (Formula (IV)) respectively.

In contrast to the conventional phosphate group-containing ProPAgens discussed above, compounds of General Formula (I) include a phosphonate group that is masked by two identical amino acid ester groups, which are enzymatically cleaved off inside cells to release an active phosphonate compound. It has been shown that the replacement of the labile -O-P- bond with a -C-C- bond resulted in a significant improvement in stability of the active phosphonate compound, which was shown to be a potent activator of Vy9/Vδ2 T-cells. Further, this activity was translated to potent lysis of bladder cancer cells *in vitro.*

As compounds of General Formula (I) have been shown to activate, with high specificity, Yγ9/Vδ2 T-cells, they are ideal candidates for development as immunotherapeutics. In particular, compounds of General Formula (I) are useful for the treatment of proliferative diseases, such as different types of cancer, osteoporosis, infectious diseases, and/or inflammatory diseases, as further outlined below.

The compounds of General Formula (I) represent a new class of small molecule activators of Vy9/Vδ2 T cells with potentially improved *in vivo* safety and efficacy. Due to their symmetrical molecular structure, the number of stereocenters is reduced, which advantageously simplifies synthesis and separation of the target compounds. This comes along with the beneficial effect that undesired product mixtures of stereoisomers having potentially different biological profiles are avoided, thereby increasing reliability of the target compounds when used in the treatment of a subject in need thereof.

General and preferred aspects of the compounds of the present invention are disclosed below and in the appended claims.

The term "halo" as used herein refers to halogen radical substituents, in particular fluoro, chloro, bromo or iodo, more suitably fluoro or chloro, and most suitable fluoro.

The term "C₂₋₂₀ alkyl" as used herein refers to a straight or branched saturated hydrocarbon chain containing from 2 to 20 carbon atoms. Examples include ethyl, n-propyl, isopropyl (iPr), n-butyl (nBu), s-butyl, t-butyl (tBu), n-hexyl, n-octyl and n-decyl.

The term "C₄₋₂₀ alkenyl" as used herein refers to a straight or branched unsaturated hydrocarbon chain containing 4 to 20 carbon atoms. Examples include butenyl, pentenyl etc.

The term "C₂₋₂₀ alcohol" as used herein refers to a straight or branched and saturated or unsaturated hydrocarbon chain containing 2 to 20 carbon atoms and including one or more hydroxyl (OH) functional group.

The term "saturated or unsaturated hydrocarbon chain" as used herein refers to a straight or branched aliphatic or aromatic hydrocarbon radical that may or may not include within said chain one or more double or triple bonds. Therefore, this term encapsulates alkyl, alkenyl alkynyl or aryl radicals. The aliphatic or aromatic hydrocarbon chain may contain a heteroatom in the chain or as (part of) a substituent.

The term "C₅₋₂₅ aryl" as used herein refers to any hydrocarbon group that contains 5 to 25 carbon atoms and includes one or more carbocyclic aromatic ring. More suitably aryl groups are C₆₋₁₄ and are preferably C₆₋₁₀ aryl groups.

The term "heteroaryl" as used herein refers to any hydrocarbon group that includes one or more aromatic ring that includes one or more heteroatom (e.g. N, O or S) as part of said ring. Particularly suitable examples of heteroaryl groups are pyridine, furan, thiophene and indole groups. A 5 to 25 membered heteroaryl group refers to a group in which the total number of ring forming atoms (carbon and heteroatom(s)) is from 5 to 25.

Other alkyl, alkenyl, aryl and/or alcohol groups are as defined but have different numbers of carbon atoms. For example, C₁₋₄ alkyl has 1 to 4 carbon atoms, such as 1, 2, 3 or 4 carbon atoms.

The hydrocarbon chain and/or the aryl, heteroaryl, alkyl, alkenyl or alcohol radicals may be optionally substituted with one or more heteroatom (e.g. O, S, or N) containing functional group. Examples of suitable heteroatom containing groups include, but are not limited to, nitro, nitrone, halo, amino, amido, cyano, carboxyl, sulphonyl, hydroxyl, alkoxy, ketone, aldehyde, thiol, thioether, and non-aromatic heterocyclic groups. The hydrocarbon chain may or may not contain a saturated or unsaturated cyclic ring(s). As used herein, any carbon number of a hydrocarbon chain, alkyl, alkenyl, aryl or alcohol radical includes any carbon atoms present in substituents. In preferred embodiments, the hydrocarbon chain and/or the aryl, heteroaryl, alkyl, alkenyl or alcohol radical are optionally substituted with one or more heteroatom containing functional group selected from thiol, thioether, alkoxy and amino (which may be a primary or secondary amino group).

Salts of the compounds of General Formula (I) are suitably pharmaceutically or veterinary acceptable salts. Depending on the nature of R1 to R6, these may be basic addition salts such as sodium, potassium, calcium, aluminium, zinc, magnesium and other metal salts as well as choline, diethanolamine, ethanolamine, ethyl diamine, megulmine and other well-known basic addition salts as summarised in Paulekuhn et al., (2007) J. Med. Chem. 50: 6665-6672 and/or known to those skilled in the art. Alternatively, when the compound of general formula (I) contains an amino group, this may be quaternised to form a salt with a counter ion such as halide, hydroxide, sulfate, nitrate, phosphate, formate, acetate, trifluoroacetate, fumarate, citrate, tartrate, oxalate, succinate, mandelate, methane sulfonate and p-toluene sulfonate.

In some compounds of General Formula (I), R2 is a C₄₋₁₆, and preferably a C₄₋₈, such as C₅₋₇, or C₄, C₅, C₆, C₇, C₈, alkyl, alkenyl or alcohol radical. More preferably, R2 is an alcohol radical as defined above.

In particularly preferred embodiments, R2 is a radical according to Formula (IV), even more preferred a radical according to Formula (IV) with R5 being OH.

As noted above, the compounds of General Formula (I) comprise a masked phosphonate group, which includes a -C(X)(Y)-P- bond, which is shown to be more stable than the -O-P-bond of conventional ProPAgens. In preferred embodiments, at least one, and more preferably both, of X and Y represent halo, preferably fluoro.

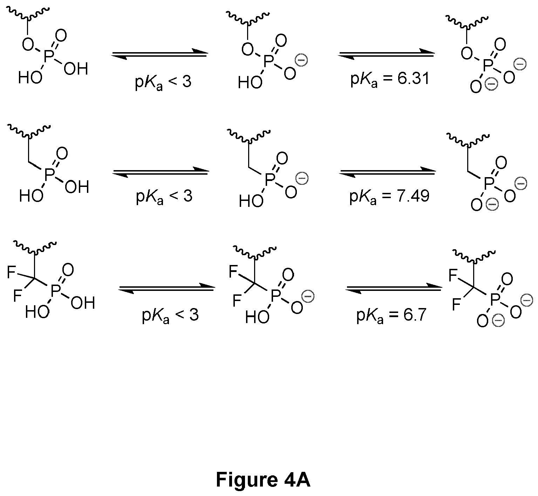

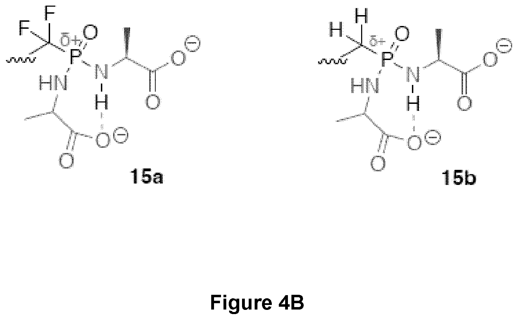

Although the change from a -O-P- bond to a -CH₂-P- bond has been shown to achieve better serum stability of the active compound, the p*K*ₐ value for the second deprotonation of the phosphonate group (p*K*ₐ = 7.49) is significantly different from that of the phosphate group (p*K*ₐ = 6.31) (Figure 4). This as a result affects the full ionization of the active compound under physiological pH (< 7.4) and hence the binding affinity to the target protein, which requires the full ionization of the phosphate group to bind a positively charged pocket (arginine-rich) on the butyrophilin 3A1 intracellular domain.

However, it has been found that, by providing a difluoromethyl phosphonate (-CF₂-P-) bond, the active compounds combine excellent stability in physiological environments with a p*K*ₐ value for the second deprotonation (6.7) very close to that of the second deprotonation of the native phosphate compound itself (Figure 4). Therefore, the mono and/or dihalomethyl, in particular mono and/or difluoromethyl phosphonate derivatives of compounds according to General Formula (I) combine excellent stability with potent activation of Vy9/V62 T-cells.

The compounds of General Formula (I) are prodrugs in which the monophosphonate group is masked by two identical amino acid ester moieties R1, which are both enzymatically cleaved off inside cells to release the active monophosphonate species.

Suitable amino acid ester masking groups include a wide variety of amino acid side chain (R3) groups. Typically, R3 represents a side chain, preferably a non-polar side chain, of an amino acid. Particularly preferred R3 groups comprise a C₁₋₄ alkyl chain. Examples of such R3 groups include -CH₃ (alanine), -CH₂CH(CH₃)₂ (leucine), -CH(CH₃)CH₂CH₃ (isoleucine) and -CH₂CH₂SCH₃ (methionine). Compounds in which R3 represents a methyl (CH₃) are particularly suitable.

Thus, R1 is an amino acid ester radical according to General Formula (II) derived from an amino acid selected from the group of L-amino acids and D-amino acids, preferably alanine, leucine, isoleucine and methionine. For example, the amino acid can be selected from L-alanine, L-leucine, L-isoleucine, and L-methionine. In other cases, the amino acid can be selected from D-alanine, D-leucine, D-isoleucine, and D-methionine. In preferred embodiments, the amino acid ester radical according to General Formula (II) is derived from an L-amino acid selected from the group of L-alanine, L-leucine, L-isoleucine or L-methionine, most preferably, the amino acid ester radical according to General Formula (II) is derived from L-alanine.

R4 is a saturated or unsaturated straight or branched and optionally substituted hydrocarbon chain and may be aliphatic or aromatic. Preferably, R4 is a C₁₋₆ alkyl or C₆₋₁₄ aryl group. In particularly preferred embodiments R4 is C₆₋₁₀ aryl group, even more preferably selected from methyl (Me), isopropyl (iPr), tert-butyl (tBu) and benzyl (Bn), and most preferably is a benzyl (Bn) group. Compounds according to General Formula (I) comprising a benzyl ester have a higher rate of degradation and improved lipophilicity (and thus improved cell uptake) in comparison with aliphatic ester-based compounds.

In preferred embodiments, R2 is a radical according to Formula (IV), preferably with R5 is OH, and R1 is an amino acid ester radical according to General Formula (II) derived from L-alanine, L-leucine, L-isoleucine or L-methionine, most preferably L-alanine, and R4 is selected from methyl, isopropyl, tert-butyl and benzyl, more preferably, wherein R4 is isopropyl or benzyl, most preferably wherein R4 is benzyl.

In other preferred embodiments, R2 is a radical according to Formula (IV), preferably with R5 is OH, and R1 is an amino acid ester radical according to General Formula (II) derived from L-alanine, L-leucine, L-isoleucine or L-methionine, most preferably L-alanine, and R4 is selected from methyl, isopropyl, tert-butyl and benzyl, more preferably, wherein R4 is isopropyl or benzyl, most preferably wherein R4 is benzyl, and both, of X and Y representing halo, preferably fluoro.

In yet preferred embodiments, in a compound according to any of the two preceding embodiments, R1 is an amino acid ester radical according to General Formula (II) derived from L-alanine.

In yet preferred embodiments, in a compound according to any of the three preceding embodiments, R2 is a radical according to Formula (IV) with R5 is OH.

In yet preferred embodiments, in a compound according to any of the four preceding embodiments R4 is benzyl (Bn).

Some particular suitable Compounds of General Formula (I) include the following compounds: 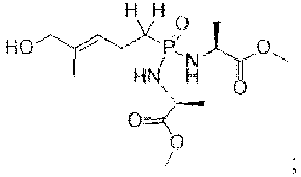 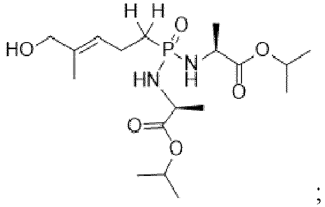 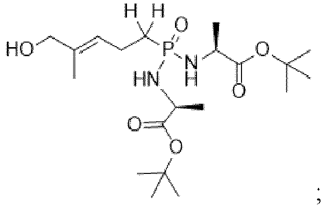 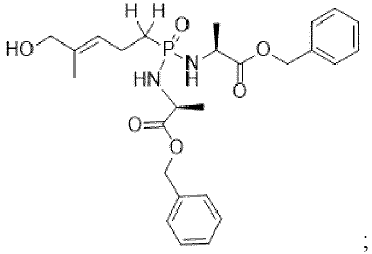 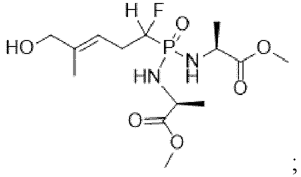 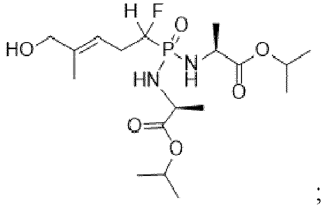 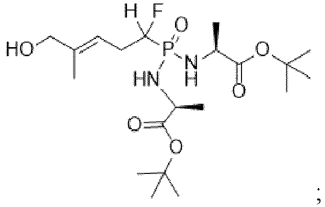 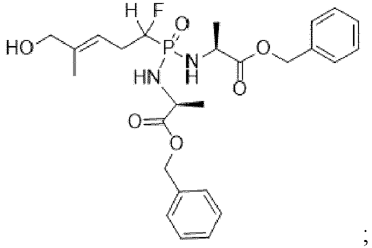 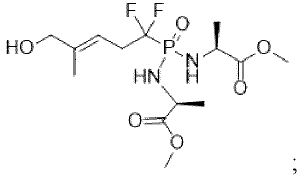 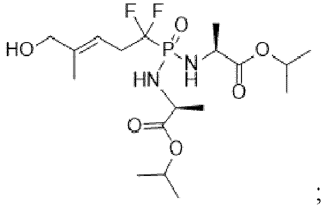 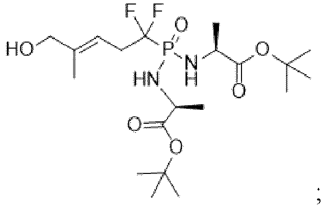 and

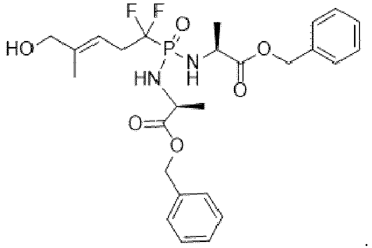

In preferred embodiments, the compound is selected from 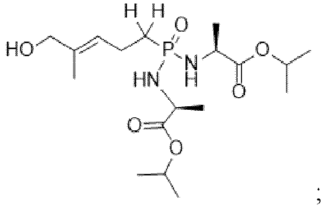 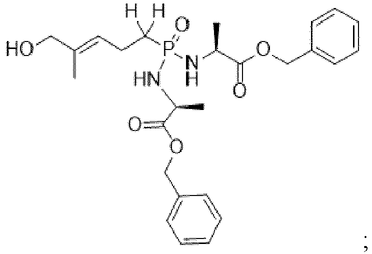 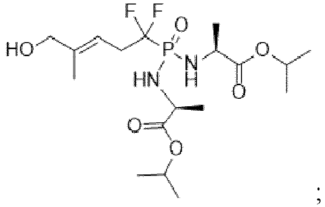 and 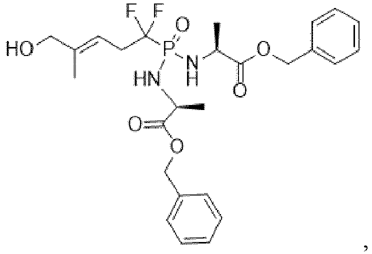 more preferably the compound is selected from: 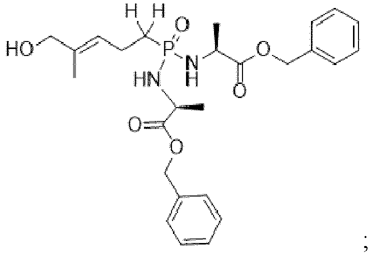 and 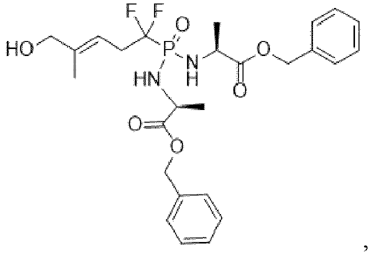 most preferably the compound is

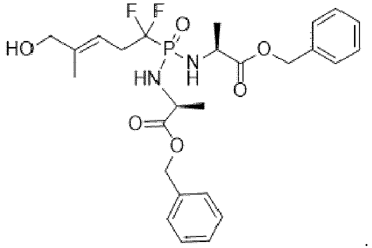

According to a second aspect, there is disclosed a compound according to General Formula (I) including all tautomers thereof: 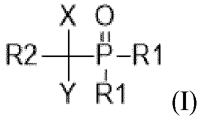 wherein R1 represents an amino acid ester radical according to General Formula (II): 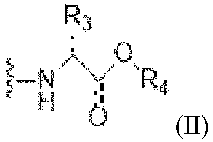 wherein R3 represents H, or a saturated or unsaturated and optionally substituted hydrocarbon chain; R4 represents a saturated or unsaturated and optionally substituted hydrocarbon chain; and wherein both R1 are identical; and

R2 represents an optionally substituted C₂₋₂₀ alkyl, C₄₋₂₀ alkenyl or C₂₋₂₀ alcohol radical; and each of X and Y independently represent H or halo, wherein at least one, and preferably both, of X and Y represent halo, preferably fluoro;
or a salt thereof.

Further and preferred aspects of compounds with at least one, and preferably both of X and Y representing halo, preferably fluoro are as described for previously disclosed embodiments herein incorporated by reference to embodiments disclosed herein.

Compounds of the present invention can be obtained by a process comprising the steps of (i) providing a phosphonic acid alkyl ester; (ii) converting the phosphonic acid alkyl ester into a phosphonic acid halogenide by removing the ester groups and subsequently subjecting to a halogenation reaction; (iii) subjecting the phosphonic acid halogenide to an esterification reaction with an amino acid ester hydrohalogenide to obtain a amino acid ester derivative; and (iv) subjecting the amino acid ester derivative of step (iii) to an olefin metathesis with prevention of alkene isomerization.

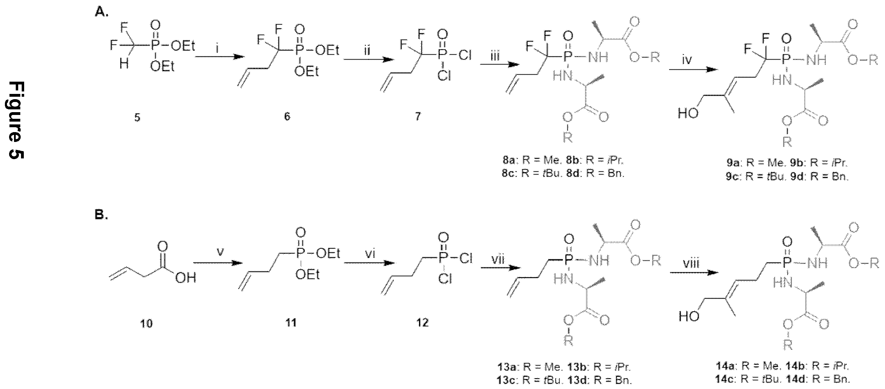

In particular, compounds of general formula (I) in which X and Y each represent F (designated herein as compounds 9a-d) are prepared via a synthetic route as summarized in Figure 5 (A) starting by reacting the commercially available α,α-difluorophosphonate 5 with allyl bromide in THF and in the presence of lithium diisopropylamine (LDA) and hexamethylphosphoramide (HMPA) to obtain compound 6 in 44% yield. Subsequently, compound 6 is treated with trimethylsilyl bromide (TMSBr) at room temperature to remove the ethoxy groups and generate the phosphonic acid derivative. This is followed by a chlorination reaction using oxalyl chloride in the presence of a catalytic amount of DMF to generate compound 7 and this is used in the next reaction without purification. Next, compound 7 is treated with 2.5 equivalents of the appropriate amino acid ester in the presence of triethylamine and this generates the desired phosphonodiamidates 8a-d in good yields (23-49%). Finally, these compounds undergo Grubbs olefin metathesis with 2-methyl-2-propenol employing the Hoveyda-Grubbs second generation catalyst in the presence of 1,4-benzoquinone to prevent alkene isomerization. This gives the desired phosphonodiamidate ProPAgens 9a-d in good yields (38-67%) in ≥95% purity.

The synthesis of compounds of general formula (I) in which X and Y each represent H (designated herein as compounds 14a-d) is summarized in Figure 5 (B) and is achieved in the same manner as that used for making 8a-d with the only exception being the preparation of compound 11. This is achieved by first reacting 3-butenoic acid (10) with oxalyl chloride in the presence of DMF to generate 3-butenoyl chloride, which is subsequently reacted with triethylphosphite to yield compound 11 in a good yield (51 %). The phosphonodiamidate ProPAgens 14a-d are obtained in good yields (33-63%) and in ≥95% purity.

It will be appreciated that the compounds of the first and second aspects of the invention may be administered as part of a pharmaceutical composition. Therefore, according to a third aspect of the invention there is provided a pharmaceutical comprising a compound of the first and/or the second aspect of the invention. The pharmaceutical composition of the invention preferably comprises a pharmaceutically acceptable excipient or carrier.

Suitable pharmaceutical excipients are well known to those of skill in the art. Pharmaceutical compositions may be formulated for administration by any suitable route, for example oral, rectal, nasal, bronchial (inhaled), topical (including eye drops, buccal and sublingual), vaginal or parenteral (including subcutaneous, intramuscular, intravenous and intradermal) administration and may be prepared by any methods well known in the art of pharmacy.

The composition may be prepared by bringing into association the compound of the first aspect of the invention with the carrier. In general, the formulations are prepared by uniformly and intimately bringing into association said compound with liquid carriers or finely divided solid carriers or both, and then if necessary shaping the product.

Formulations for oral administration in the present invention may be presented as: discrete units such as capsules, sachets or tablets each containing a predetermined amount of the compound; as a powder or granules; as a solution or a suspension of the compound in an aqueous liquid or a non-aqueous liquid; or as an oil-in-water liquid emulsion or a water-in-oil liquid emulsion; or as a bolus etc.

For compositions for oral administration (e.g. tablets and capsules), the term "acceptable carrier" includes vehicles such as common excipients e.g. binding agents, for example syrup, acacia, gelatin, sorbitol, tragacanth, polyvinylpyrrolidone (Povidone), methylcellulose, ethylcellulose, sodium carboxymethylcellulose, hydroxypropylmethylcellulose, sucrose and starch; fillers and carriers, for example corn starch, gelatin, lactose, sucrose, microcrystalline cellulose, kaolin, mannitol, dicalcium phosphate, sodium chloride and alginic acid; and lubricants such as magnesium stearate, sodium stearate and other metallic stearates, glycerol stearate, stearic acid, silicone fluid, talc waxes, oils and colloidal silica. Flavouring agents such as peppermint, oil of wintergreen, cherry flavouring and the like can also be used. It may be desirable to add a colouring agent to make the dosage form readily identifiable. Tablets may also be coated by methods well known in the art.

A tablet may be made by compression or moulding, optionally with one or more accessory ingredients. Compressed tablets may be prepared by compressing in a suitable machine the compound in a free-flowing form such as a powder or granules, optionally mixed with a binder, lubricant, inert diluent, preservative, surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active agent.

Other formulations suitable for oral administration include lozenges comprising the active agent in a flavoured base, usually sucrose and acacia or tragacanth; pastilles comprising the active agent in an inert base such as gelatin and glycerin, or sucrose and acacia; and mouthwashes comprising the active agent in a suitable liquid carrier.

Parenteral formulations will generally be sterile.

For topical application to the skin, the composition may be made up into a cream, ointment, jelly, solution or suspension etc. Cream or ointment formulations that may be used for the drug are conventional formulations well known in the art, for example, as described in standard textbooks of pharmaceutics such as the British Pharmacopoeia.

In a preferred embodiment of this aspect of the invention the composition is formulated for oral delivery.

The precise amount of a composition as defined herein which is therapeutically effective, and the route by which such compound is best administered, is readily determined by one of ordinary skill in the art. Such amounts will depend, of course, on the particular condition being treated, the severity of the condition, the individual patient parameters including age, physical condition, size and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment. It will be understood by those of ordinary skill in the art, however, that a patient may insist upon a lower dose or tolerable dose for medical reasons, psychological reasons or for virtually any other reasons.

The doses of the compound or composition according to the invention administered to a subject can be chosen in accordance with different parameters, in particular in accordance with the mode of administration used and the state of the subject. Other factors include the desired period of treatment. In the event that a response in a subject is insufficient at the initial doses applied, higher doses (or effectively higher doses by a different, more localized delivery route) may be employed to the extent that patient tolerance permits.

As mentioned above, the compounds of the first and second aspects of the invention represent prodrugs of highly stable potent activators of Yγ9/Vδ2 T-cells. Therefore, according to a fourth aspect of the invention there is provided a compound of the first and/or second aspect or a pharmaceutical composition according to the third aspect for use in medicine, and more preferably in immunotherapy. In a preferred embodiment, the immunotherapy comprises the activation of T-cells, preferably γδ T cells, even more preferably Yγ9Vδ2 T-cells. In another preferred embodiment, the immunotherapy comprises is directed to the treatment of a proliferative disease, an infectious disease, an inflammatory disease and/or osteoporosis. The immunotherapy will normally comprise the administration of a therapeutically effective amount of the compound of the invention to a subject in need thereof.

The compounds of the invention can be administered to a subject in need of treatment, e.g. a subject suffering from of a proliferative disease, such as cancer, an infectious disease, an inflammatory disease and/or osteoporosis. Upon administration, the compounds lead to the activation of T cells in the subject's body, preferably γδ T cells, and more preferably Yγ9Vδ2 T-cells. The activated T cells exert potent effector responses, such as cytotoxicity, which then directs the immune response to the respective target cells, e.g. the tumor cells. The subject can be a mammalian subject, preferably a human subject.

The immunotherapy may further comprise the administration of T cells, preferably γδ T cells, and preferably Yγ9Vδ2 T-cells. The cells may be autologous which means that they originate from the patient to be treated and have been obtained from that patient before initiation of the immunotherapy. Alternatively, the cells may be allogeneic in which case the cells have been obtained from a donor, such as from a blood sample of a blood donor. The administration of the autologous or allogeneic T cells may be performed before, after or simultaneously with administration of the ProPAgens of the present invention.

The immunotherapy may also comprise the administration of an interleukin, preferably interleukin-2 (IL-2) and more preferably human IL-2. Again, the administration of the autologous or allogeneic T cells may be performed before, after or simultaneously with administration of the ProPAgens of the present invention.

In one embodiment, the immunotherapy is directed to the treatment of a proliferative disease. Preferably, the proliferative disease is a cancer disease. The cancer disease to be treated with the compounds or pharmaceutical compositions of the invention is not limited any includes hematologic and non-hematologic cancers. In one preferred embodiment, the immunotherapy is directed to the treatment of leukemia, i.e. a malignant disease of the blood-forming tissues, such as the bone marrow or the lymphatic system. The leukemia to be treated with the compounds or pharmaceutical compositions of the invention can include any subclass of leukemia, including acute lymphoblastic leukemia (ALL), chronic lymphocytic leukemia (CLL), acute myelogenous leukemia (AML) and chronic myelogenous leukemia (CML). In one embodiment, the immunotherapy is directed to the treatment of ALL, and more particularly to the treatment of precursor B acute lymphoblastic leukemia, precursor T acute lymphoblastic leukemia, Burkitt's leukemia, or acute biphenotypic leukemia. In another embodiment, the immunotherapy is directed to the treatment of CLL, and more particularly to the treatment of B-cell prolymphocytic leukemia. In yet another embodiment, the immunotherapy is directed to the treatment of hairy cell leukemia (HCL), T-cell prolymphocytic leukemia (T-PLL), large granular lymphocytic (LGL) leukemia, clonal eosinophilias or adult T-cell leukemia.

Alternatively, the immunotherapy can be directed to the treatment of a solid tumor. The tumor to be treated with the compounds or pharmaceutical compositions of the invention includes, but is not limited to, bladder cancer, prostate cancer, lung cancer, neck cancer, skin cancer, breast cancer, colon cancer, liver cancer, pancreatic cancer, ovarian cancer, brain cancer, stomach cancer, kidney cancer, uterus cancer, bone cancer, oesophagus cancer, Kaposi's sarcoma, oropharyngeal cancer, testicular cancer, thyroid cancer, lymphoma, adrenocortical cancer, craniopharyngioma, cutaneous T-Cell lymphoma, ductal carcinoma in situ, endometrial cancer, ependymoma, cardiac tumors, cholangiocarcinoma, germ cell tumors, embryonal tumors, lip cancer, oral cancer, multiple myeloma, small intestine cancer, nasal cancer, sinus cancer, anal cancer, Burkitt lymphoma, bile duct cancer, cervical cancer, laryngeal cancer, Hodgkin lymphoma, non-Hodgkin lymphoma, Wilms' tumor, plasma cell myeloma, retino-blastoma or mesothelioma.

In a particularly preferred embodiment, the cancer to be treated with the immunotherapy of the invention is selected from the group consisting of a hematologic cancer, such as leukemia, bladder cancer, prostate cancer, lung cancer, neck cancer, skin cancer, breast cancer and mesothelioma.

In another embodiment, the immunotherapy is directed to the treatment of an infectious disease. Suitable infections for treatment with the compounds or pharmaceutical compositions of the invention include bacterial, viral and fungal infections. Particularly suitable infections are bacterial infections, such as infections by pathogenic bacteria of the genus *Mycobacterium,* including *M. africanum, M. canettii, M. caprae, M. bovis, M. microti, M. mungi, M. pinnipedii*, *M. tuberculosis, M. genavense; M. leprae; M. immunogenicum; M. conspicuum; M. mucogenicum; M. ulcerans; M. xenopi, M. shottsii, M. avium and M. paratuberculosis.* Thus in a preferred embodiment, the immunotherapy of the present invention is directed to the treatment of tuberculosis or leprosy.

The compounds or pharmaceutical compositions of the invention can also be used for treating bacterial infections of the genus *Salmonella,* including the species *S. enteric,* and preferably one of the subspecies *S. enterica subsp. enterica, S. enterica ssp. arizonae* and *S. enterica ssp. diarizonae.* In a particularly preferred embodiment, compounds of the invention can also be used for treating bacterial infections with a pathogenic *S. enterica subsp. enterica* strain that causes typhoid. Therefore, the compounds of General Formula (I) are particularly useful for the treatment of typhoid.

In another embodiment, the immunotherapy is directed to the treatment of a fungal infection. Fungal infections that can be treated with the compounds and pharmaceutical compositions of the invention include infections with a fungus of the genus *Aspergillus,* in particular lung infections. In one embodiment, the infection to be treated is an aspergillosis caused by a species selected from *A. fumigatus, A. flavus, A. terreus, A. nidulans* and *A. niger.* Infections with a fungus of the genus *Blastomyces* can also be treated by the compounds and pharmaceutical compositions of the invention. In one embodiment, the infection to be treated is a blastomycosis caused by the species *B. dermatitidis.* Also treatable are infections with a fungus of the genus *Candida,* in particular invasive infections. In one embodiment, the infection to be treated is a candidiasis caused by a species selected from C. *albicans, C. auris, C. blankii, C. stellatoidea, C. dubliniensis, C. famata, C. glabrata, C. krusei, C. parapsilosis and C. tropicalis.* Also treatable are infections with a fungus of the genus *Cryptococcus.* In one embodiment, the infection to be treated is a cryptococcosis caused by a species selected from C. *neoformans* and C. *gattii.* The compounds and pharmaceutical compositions of the invention are also suitable to treat fungal infections of the nail, skin or eye.

In yet another embodiment, the immunotherapy is directed to the treatment of a viral infection. The type of viral infection that can be treated with the compounds and pharmaceutical compositions of the invention include viral infections caused by a wide range of RNA and DNA viruses, including, orthomyxoviruses, paramyxoviruses, flavivirus, pestivirus, hepavirus, arenaviruses, herpes viruses, adenoviruses, poxviruses, and retroviruses. In another embodiment, the immunotherapy is directed to the treatment of parasitic infections. Particularly suitable parasitic infections that can be treated with the compounds or pharmaceutical compositions of the invention include infections with organisms of the genus *Plasmodium,* such as *P. vivax, P. falciparum, P. malariae, P. ovale* and *P. knowlesi.* In a particularly preferred embodiment, compounds of the invention or pharmaceutical compositions comprising same are used for treating a *Plasmodium* species that causes malaria. Therefore, the compounds of General Formula (I) or pharmaceutical compositions comprising same are particularly useful for the treatment of malaria. Other suitable parasitic infections that can be treated include infections with organisms of the genus *Toxoplasma,* such as *T. gondii.* In a particularly preferred embodiment, compounds of the invention are used for treating a *Toxoplasma* species that causes toxoplasmosis. Therefore, the compounds of General Formula (I) or pharmaceutical compositions comprising same are particularly useful for the treatment of toxoplasmosis.

In one embodiment, the immunotherapy is directed to the treatment of an inflammatory disease. Hence, the compounds of the invention or pharmaceutical compositions comprising same are useful for treating diseases or disorders that are characterized by a deregulated immune status or an excessive inflammatory reaction, in particular chronic inflammatory diseases. Such diseases or disorders include, for example, autoimmune diseases and hypersensitivity reactions. The inflammatory disease to be treated may be local or systemic. The type of inflammatory diseases or condition that benefit from treatment is not limited and includes, but is not limited to arterial occlusive diseases, such as peripheral artery occlusive disease (pAOD), critical limb ischaemia, arteriosclerosis, cerebral infarction, myocardial infarction, renal infarction, intestinal infarction, angina pectoris, and other conditions caused by arterial occlusion or constriction; inflammation associated systemic with metabolic disorders, including Type II diabetes and obesity-related metabolic syndrome; dermatological diseases, including eczema. In a preferred embodiment, the inflammatory disease is an autoimmune disease. The type of autoimmune disease to be treated is not limited and includes ulcerative colitis, Crohn's disease, rheumatoid arthritis, autoimmune cardiomyopathy, autoimmune hepatitis, lupus erythematosus, Graves' disease, Guillain-Barré syndrome (GBS), Hashimoto's thyroiditis, idiopathic thrombocytopenic purpura, juvenile idiopathic arthritis, myasthenia gravis, pemphigus vulgaris, psoriasis, Reiter's syndrome, scleroderma, Sjögren's syndrome, vasculitis, vitiligo, and Wegener's granulomatosis.

In another embodiment, the immunotherapy is directed to the treatment of a hypersensitivity reaction. For example, the hypersensitivity reaction may be selected from the group of asthma, eczema, allergic rhinitis, angioedema, drug hypersensitivity, and mastocytosis.

In another embodiment, the immunotherapy is directed to the treatment of osteoporosis. The osteoporosis to be treated can be a primary or secondary osteoporosis.

Also provided by the fourth aspect of the invention is a method of immunotherapy comprising administering a therapeutically effective amount of a compound of the first or second aspect of the invention or a pharmaceutical composition of the third aspect of the invention to a subject in need thereof, such as a human subject. By delivering the compound of the first or second aspect of the invention or a pharmaceutical composition of the third aspect of the invention to a subject to be treated, the γδ T cells of the subject are activated *in vivo* which forms an immune response against the respective target cells, e.g. cancer cells. The method of treatment can be directed to the treatment of any of the conditions or diseases mentioned above, i.e. the treatment of a proliferative disease, an infectious disease, an inflammatory disease and/or osteoporosis. The method of treatment may comprise the administration of autologous or allogeneic T-cells, preferably γδ T cells, and more preferably Yγ9Vδ2 T-cells, as mentioned elsewhere herein. The immunotherapy may also further comprise the administration of an interleukin, preferably IL-2 and more preferably human IL-2.

In a fifth aspect, the invention relates to an *ex vivo* method of preparing a population οf γδ T cells for therapeutic use, said method comprising:
(i) providing a population οf γδ T cells;
(ii) culturing the population of γδ T cells in the presence of a compound according to the first or fifth aspect of the invention as set out above.

The method comprises the contacting of yδ T cells with the compounds of the invention in order to induce their activation and/or expansion. In the first step of the method a population of γδ T cells is provided. The population of γδ T cells can be obtained from various sources, such as a blood sample from a donor or the patient to be treated. The γδ T cells can be obtained from peripheral blood mononuclear cells (PBMCs) isolated from peripheral blood or from cord blood mononuclear cells (CBMC) isolated from umbilical blood. In the second step of the method, the population οf γδ T cells is cultured in the presence of a compound according to the first or second aspect of the invention for a time period and under conditions that result in their activation and/or expansion.

According to a sixth aspect, the invention relates to a population οf γδ T cells obtained by the method according to the fifth aspect. The cells that have been treated by the above method according to the fifth aspect of the invention are preferably Yγ9/Vδ2 T cells and can be advantageously used in medicine, and in particular in immunotherapy.

The invention, according to a seventh aspect, also provides a pharmaceutical composition comprising the population of γδ T cells according to the sixth aspect of the invention. For these pharmaceutical compositions, the explanations set out above in connection with the pharmaceutical compositions of the third aspect of the invention apply accordingly.

According to an eighth aspect, the invention relates to the population of yδ T cells according to the sixth aspect of the invention for medical use, and in particular for use in immunotherapy. Preferably, said immunotherapy is directed to the treatment of a proliferative disease, an infectious disease, an inflammatory disease and/or osteoporosis. For the medical use of the cells according the eighth aspect of the invention, the explanations set out above in connection with the fourth aspect of the invention apply accordingly. Accordingly, the γδ T cells according to the sixth aspect of the invention can be used for treating the same conditions and diseases that have been discussed herein in relation to the fourth aspect of the invention. In other words, the γδ T cells according to a sixth aspect of the invention, which have been obtained by the method of the fifth aspect of the invention, can be used for treating a proliferative disease, an infectious disease, an inflammatory disease and/or osteoporosis.

When using the cells according to a sixth aspect of the invention according to the eighth aspect of the invention, the treatment can be an autologous treatment. This means that the γδ T cells which are subjected to the method of the fifth aspect of the invention have been obtained from the patient that is ultimately treated with the γδ T. In this case, γδ T cells are obtained from the patient, subsequently treated by the method of the fifth aspect of the invention, and subsequently reintroduced into the patient. Alternatively, the treatment can be an allogeneic treatment which means that the γδ T cells which are subjected to the method of the fifth aspect of the invention have been obtained from a donor which is not the patient that is ultimately treated.

According to a ninth aspect, the invention relates to a compound of the first or second aspect of the invention in the preparation of a pharmaceutical composition for immunotherapy. As outlined elsewhere herein, said immunotherapy is preferably directed to the treatment of a proliferative disease, an infectious disease, an inflammatory disease and/or osteoporosis.

Throughout the description and claims of this specification, the words "comprise" and "contain" and variations of the words, for example "comprising" and "comprises", mean "including but not limited to" and do not exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

All references, including any patent or patent application, cited in this specification are hereby incorporated by reference. No admission is made that any reference constitutes prior art. Further, no admission is made that any of the prior art constitutes part of the common general knowledge in the art.

Preferred features of each aspect of the invention may be as described in connection with any of the other aspects.

Other features of the present invention will become apparent from the following examples. Generally speaking, the invention extends to any novel one, or any novel combination, of the features disclosed in this specification (including the accompanying claims and drawings). Thus, features, integers, characteristics, compounds or chemical moieties described in conjunction with a particular aspect, embodiment or example of the invention are to be understood to be applicable to any other aspect, embodiment or example described herein, unless incompatible therewith.

Moreover, unless stated otherwise, any feature disclosed herein may be replaced by an alternative feature serving the same or a similar purpose.

The Invention will now be described by way of example only with reference to the Examples below and to the following Figures:
**Figure 1** shows the chemical structures of reported small molecule Vy9/V62 T-cells activators: Naturally-occurring PAgs (*E*)-4-hydroxy-3-methylbut-2-enyl pyrophosphate (HMBPP) and isopentenyl pyrophosphate (IPP); Synthetic molecules risedronate and zoledronate.
**Figure 2** shows the application of the aryloxy triester phosphoramidate prodrug technology to the monophosphate derivative of HMBPP (HMBP). The monophosphate group is masked by an aryl group and an amino acid ester, which are both enzymatically cleaved off inside cells to release the active monophosphate species. Instability was observed due to the cleavage of the -P-O- bond of these compounds (shaded).
**Figure 3A** shows the general chemical structure of aryloxy diester phosphonamidate ProPAgens according to Mehellou et al. 2022.
**Figure 3B** shows the general chemical structure of symmetrical amino acid ester-derived phosphonodiamidate ProPAgens according to the present invention.
**Figure 4A** shows the pKa values of phosphate and different phosphonate groups.
**Figure 4B** shows the chemical structures of metabolite 15 showing the electron withdrawing effects of the fluorine atoms in the difluoromethylene phosphonate, which make the phosphorous center a stronger electrophile (15a) compared to the methylene phosphonate 15b. This also has an effect on the strength if the possible H-intramolecular bond which is stronger in 15b than in 15a due to the fluorine's withdrawing effects. This makes the cyclisation of the methylene phosphonates potentially slower than their non-fluorinated counterparts.
**Figure 5** shows the synthesis of symmetrical amino acid ester-derived_phosphonodiamidate prodrugs of (A) HMBP methylphosphonate (14a-d); and (B) HMBP difluoromethylphosphonate (9a-d). Reagents and conditions: A. (i) LDA, HMPA, THF, allyl bromide, -78 °C, yield 44%; (ii) TMSBr, DCM, 50 °C, N₂, 3 h then (COCl)₂, DMF cat, DCM, rt, 2 h; (iii) L-alanine ester hydrochloride, DCM, N₂, -78 °C, TEA then rt, overnight, yield: 23-49%; (iv) 2-methyl-2-propenol, 1,4-benzoquinone, Hoveyda-Grubbs catalyst 2^{nd} generation, DCM, 78 °C, yields: 38-67%. B. (v) oxalyl chloride (solvent and reagent), N₂, 0 °C to rt, DMF then (EtO)₃P, 0 °C to rt, overnight, yield 51%; (vi), TMSBr, DCM, 50 °C, N₂, 3 h then (COCl)₂, DMF cat, DCM, rt, 2 h; (vii) L-alanine ester hydrochloride, DCM, N₂, -78 °C, TEA then rt, overnight, yield: 23-44%; (vii) 2-methyl-2-propenol, 1,4-benzoquinone, Hoveyda-Grubbs catalyst 2^{nd} generation, DCM, 78 °C, yields: 33-63%.
**Figure 6** shows the stability of the HMBP phosphonodiamidate ProPAgen 9b in human serum at 37 °C for 7.5 hours as monitored by ³¹P NMR.
**Figure 7A** shows the *in vitro* Activation of human Yγ9/Vδ2⁺ T cells by ProPAgens 9a-d. HMBP ProPAgens of the present invention were used at concentrations of 10pM to 100µM, and then at 1aM to 100µM for compound 9d in a separate experiment, see Figure 7E.
**Figure 7B** shows the *in vitro* Activation of human Yγ9/Vδ2⁺ T cells by ProPAgens 14a-d. HMBP ProPAgens of the present invention were used at concentrations of 10pM to 100µM, and then at 1aM to 100µM for compound 14d in a separate experiment, see Figure 7E.
**Figure 7C** shows the *in vitro* Activation of human Yγ9/Vδ2⁺ T cells by Zoledronate and HMBPP at concentrations of 10pM to 100µM. Zoledronate and HMBPP were used as prior art compounds for comparison with HMBP ProPAgens according to the present invention.
**Figure 7D** shows the EC₅₀ values were calculated using GraphPad Prism v9 based on the results of the activation assay. CLogP values were calculated using ChemDraw Professional 16.0. The EC₅₀ value (nM) for 14a was not determined as it was not possible to obtain an accurate potency level.
**Figure 7E** shows the phosphonodiamidate ProPAgen 9d- and 14d-mediated activation of Vy9/Vδ2 T cells after overnight incubation of PBMC with 9d at 1 attomolar (aM) to 100µM and 14d at 1 femtomolar (fM) to 100µM in order to determine the EC50 values of these compounds. The level of activation is presented as % CD69+ CD25+ Vy9/V62 T cells. Data was analyzed with GraphPad Prism v9 software and is shown as mean ± SE (n=4).

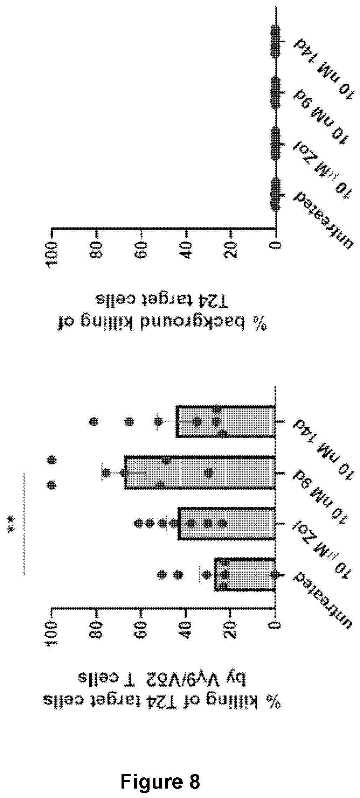

**Figure 8** shows the cytotoxocity assay showing potent lysis of bladder cancer cells following incubation with the HMBP phosphonate ProPAgens 14d and 9d. ProPAgen 14d and 9d mediate the specific lysis of T24 urinary bladder carcinoma cells by Vy9/V62 T-cells. % Killing of T24 cells was calculated with this formula: [(experimental release-spontaneous release)/(maximal release -spontaneous release)]x100. Data is shown as mean ± SE (n = 7). Statistical analysis was performed using one-way ANOVA and Tukey's multiple comparisons test on GraphPad Prism v9. **p < 0.0063. A positive control for cell death (target cells incubated with 10% v/v DELFIA lysis buffer) and media-only treated control (no drug) were also included.
**Figure 9** shows the phosphonodiamidate ProPAgen 9d- and 14d-mediated activation of CD8+ T cells (left) in the same overnight cultures as Vy9/V62 T cells (mean ± SE; n=4). Level of Vy9/Vδ2 T cell activation with DMSO (right) diluted in the same way as the ProPAgens (mean ± SE; n=4). Data was analyzed with GraphPad Prism v9 software.
**Figure 10A** shows the postulated mechanism of phosphonodiamidate prodrugs as suggested by McGuigan et al. 2018.
**Figure 10B** shows the *in vitro* carboxypeptidase Y-mediated breakdown of the phosphonodiamidate ProPAgen 9b. ³¹P-NMR spectrum of ProPAgen 9b alone and at different time points (as shown), following incubation with recombinant carboxypeptidase Y at 37 °C for 6.5 h.

### EXAMPLES

The present invention is further described in more detail by the following examples which are only provided for illustrating the invention and which are not to be construed as limiting the scope of the invention. The following material and methods were used in the Examples.

### Example 1: Synthesis of aryloxy phosphoramidate ProPAgens of HMBP methylphosphonates

All reagents and solvents were of general purpose or analytical grade and were purchased from Sigma-Aldrich Ltd., Fisher Scientific, Fluorochem or Acros. ³¹P, ¹H, ¹⁹F and ¹³C NMR data were recorded on a Bruker Avance DPX500 spectrometer operating at 202, 500 and 125 MHz. Chemical shifts (δ) are quoted in ppm, and *J* values are quoted in Hz. In reporting spectral data, the following abbreviations were used: *s* (singlet), *d* (doublet), *t* (triplet), *q* (quartet), *dd* (doublet of doublets), *td* (triplet of doublets), and *m* (multiplet). All of the reactions were carried out under nitrogen atmosphere and were monitored with analytical thin layer chromatography (TLC) on precoated silica plates (kiesel gel 60 F254, BDH). Compounds were visualized by illumination under UV light (254 nm) or by the use of KMnO₄ stain followed by heating. Flash column chromatography was performed with silica gel 60 (230-400 mesh) (Merck). HPLC was carried out on a SHIMADZU Prominence-i quaternary low-pressure gradient pump with a Prominence-i UV detector (190 to 700 nm). All solvents for HPLC were HPLC grade purchased from Fisher Scientific. HPLC data analysis was performed using the SHIMADZU Lab solutions software package. The purity of the tested ProPAgens was determined by HPLC, and they were all of ≥95% purity.

**Diethyl(1,1-difluorobut-3-en-1-yl)phosphonate (6):** To a solution of lithium diisopropylamine (LDA) (1.0 M in hexane/THF, 7.97 mL, 1 eq., 7.97 mmol) and hexamethylphosphorramide (HMPA) (1.38 mL, 1 eq., 7.97 mmol) in 5 mL THF at -78 °C was added a cooled solution of diethyl α,α-difluorophosphonate (1.7 mL, 1 eq., 7.97 mmol) in 3 mL THF. After stirring for 2 min, allyl bromide (5 mL, 1.2 eq., 9.56 mmol) was added quickly under fierce stirring. After 10 min, the reaction was quenched by NH4Cl and extracted by diethyl ether (10 mL) and ethyl acetate (2×20 mL). The combined organic phase was dried over MgSO4 and concentrated under reduced pressure. The crude was separated by flash column chromatography (EtOAc: Hexane 4:6). Yield: 793 mg (44 %). ¹H NMR (500 MHz, CDCl3): 5.83-5.91 (m, 1H, CH=CH₂), 5.31 (s, 1H, CH=CH₂, trans), 5.29 (d, *J =* 5.05 Hz, 1H, CH=CH₂, cis), 4.26-4.32 (m, 4H, 2 × CH₂CH₃), 2.80-2.91 (m, 2H, CH₂CH=CH₂), 1.40 (t, *J=* 7.07 Hz, 6H, 2 × CH₂CH₃). ¹³C NMR (125 MHz, CDCl3): 127.0, 126.9, 121.34, 64.42 (d, *J =* 6.87 Hz), 38.49-38.96 (m), 16.39 (d, *J =* 5.48 Hz). ³¹P NMR (202 MHz, CDCl3): 6.94 (t, *J =* 107.43 Hz). ¹⁹F NMR (470 MHz, CDCl₃): -111.23 (d, J = 108.42 Hz).

**Diethyl but-3-enoylphosphonate (11):** This product was synthesized over two steps. First, 3-butenoic acid (1.5 mL, 1 eq., 6 mmol) and oxalyl chloride (1 mL, 2 eq., 12 mmol) were added to a round bottom flask under nitrogen inert atmosphere. The mixture was cooled down to 0 °C and three drops of DMF were added to catalyze the reaction. The mixture was allowed to warm up to room temperature and monitored by TLC. When starting materials spot disappeared, the excess oxalyl chloride was removed under reduced pressure and the crude, 3-butenoyl chloride, was used for the next step without purification. In the second step, a dry round bottom flask was charged with the crude 3-butenoyl chloride from the previous step and cooled down to 0 °C. Then, triethylphosphite was added dropwise. The mixture was allowed to warm up to room temperature and stirred overnight. After removing solvents under reduced pressure, the crude was purified by column chromatography (EtOAc: Hexane 1:1). Yield: 626 mg (51%). ¹H NMR (500 MHz, CDCl₃): 5.92-6.03 (m, 1H, CH=CH₂), 5.26-5.31 (m, 2H, CH=CH₂), 4.08-4.22 (m, 4H, 2 × CH₂CH₃), 3.31 (dt, *J* = 6.95, 1.43, 2H, COCH₂), 1.35 (t, *J* = 7.08, 6H, 2 × CH₂CH₃). ¹³C NMR (125 MHz, CDCl₃): 173.90, 134.92, 119.53, 62.84, 38.54, 16.20. ³¹P NMR (202 MHz, CDCl₃): 9.07 (s).

**General procedure for synthesizing 7 and 12.** These compounds were synthesized over two steps. First, compounds 6 or 11 (1 eq.) was dissolved in 5 mL DCM and TMSBr (10 eq.) was added under nitrogen inert atmosphere. The reaction was refluxed at 50 °C for 3 h, and then the solvents and excess TMSBr were evaporated under reduced pressure. The crude product was then chlorinated by dissolving in 10 mL DCM and three drops of DMF were added as a catalyst under nitrogen inert atmosphere. After that, oxalyl chloride (10 eq.) was added dropwise. The reaction was stirred at room temperature for 2 h and solvents and excess oxalyl chloride were removed under reduced pressure. The crude product, 7 or 12, was used in the next step without further purification.

**General procedure for synthesizing 8a-d and 13a-d.** Compounds 7 or 12 18 (1 eq.) were dissolved in 10 mL DCM under nitrogen inert atmosphere along with the appropriate L-alanine ester hydrochloride (2.5 eq.). The mixtures were then cooled down to -78 °C and TEA (4 eq.) was added dropwise. The reactions were allowed to warm up to room temperature and stirred overnight. The solvent was removed under reduced pressure and the crude was dissolved in EtOAc. Upon filtration, the filtrate was concentrated and the crude product was purified by flash column chromatography (EtOAc: Hexane 1:1) to give the desired product.

**Dimethyl 2,2'-(((1,1-difluorobut-3-en-1-yl)phosphoryl)bis(azanediyl))(2S,2'S)-dipropionate (8a).** Yield: 144 mg (36%). ¹H NMR (500 MHz, CDCl₃): 5.81-5.89 (m, 1H, CH=CH₂), 5.26-5.31 (m, 2H, CH=CH₂), 4.05-4.15 (m, 2H, 2×NHCH), 3.75 (d, *J* = 4.15 Hz, 6H, 2×OCH₃), 3.53 (t, *J* = 10.57 Hz, 1H, NH), 3.34 (t, *J* = 11.70 Hz, 1H, NH), 2.85 (tt, *J* = 6.40 Hz, 29.09 Hz, 2H, CH₂CF₂), 1.44 (t, *J* = 7.20 Hz, 6H, 2×NHCHCH₃). ¹³C NMR (125 MHz, CDCl₃): 176.6, 127.4, 121.5, 52.6, 48.8, 38.1, 21.6. ³¹P NMR (202 MHz, CDCl₃): 12.82 (t, *J*= 95.58 Hz). ¹⁹F NMR (470 MHz, CDCl₃): -110.08 (d, *J*= 96.29 Hz), -110.66 (d, *J=* 54.14 Hz), -110.86 (d, *J*= 54.18 Hz), -111.44 (d, *J=* 96.12 Hz).

**Diisopropyl 2,2'-(((1,1-difluorobut-3-en-1-yl)phosphoryl)bis(azanediyl))(25,2'S)-dipropi-onate (8b).** Yield: 209.4 mg (49%). ¹H NMR (500 MHz, CDCl₃): 5.81-5.89 (m, 1H, CH=CH₂), 5.27-5.30 (m, 2H, CH=CH₂), 5.01-5.06 (m, 2H, OCH(CH₃)₂), 3.99-4.12 (m, 2H, 2×NHCH), 3.57 (t, *J* = 10.57 Hz, 1H, NH), 3.34 (t, *J* = 11.50 Hz, 1H, NH), 2.85 (tt, *J* = 6.23 Hz, 28.97 Hz, 2H, CH₂CF₂), 1.40-1.44 (m, 6H, 2×NHCHCH₃), 1.24-1.28 (m, 12H, OCH(CH₃)₂). ¹³C NMR (125 MHz, CDCl₃): 173.3, 127.5, 121.4, 69.2, 48.9, 37.9, 21.6, 21.9. ³¹P NMR (202 MHz, CDCl₃): 12.78 (t, J = 95.37 Hz). ¹⁹F NMR (470 MHz, CDCl₃): -110.18 (d, *J* = -110.18 Hz), -110.74 (d, *J* = 31.24 Hz), -110.94 (d, *J* = 30.16 Hz), -111.49 (d, *J* = 94.82 Hz).

**Di-tert-butyl 2,2'-(((1,1-difluorobut-3-en-1-yl)phosphoryl)bis(azanediyl))(2S,2'S)-dipro-pionate (8c).** Yield: 113 mg (25%). ¹H NMR (500 MHz, CDCl₃): 5.81-5.89 (m, 1H, CH=CH₂), 5.26-5.29 (m, 2H, CH=CH₂), 3.92-4.01 (m, 2H, 2×NHCH), 3.54 (t, *J* = 10.65 Hz, 1H, NH), 3.31 (t, *J* = 11.52 Hz, 1H, NH), 2.84 (tt, *J* = 6.01 Hz, 29.49 Hz, 2H, CH₂CF₂), 1.46 (d, *J* = 4.95 Hz, 18H, 2×OC(CH₃)₃), 1.39 (t, *J* = 7.47 Hz, 6H, 2×NHCHCH₃). ¹³C NMR (125 MHz, CDCl₃): 173.0, 127.5, 121.3, 82.0, 49.4, 37.9, 27.9, 21.7. ³¹P NMR (202 MHz, CDCl₃): 12.80 (t, *J* = 94.97 Hz). ¹⁹F NMR (470 MHz, CDCl3): -110.35 (d, *J* = 94.45 Hz), -110.89 (d, *J* = 11.55 Hz), -111.09 (d, *J* = 11.95 Hz), - 111.62 (d, *J* = 96.38 Hz).

**Dibenzyl 2,2'-(((1,1-difluorobut-3-en-1-yl)phosphoryl)bis(azanediyl))(2S,2'S)-dipropion-ate (8d).** Yield: 122 mg (23%). ¹H NMR (500 MHz, CDCl₃): 7.30-7.37 (m, 10H, Ph), 5.77-5.82 (m, 1H, CH=CH₂), 5.22-5.27 (m, 2H, CH=CH₂), 4.11-4.16 (m, 2H, 2×NHCH), 3.52 (t, *J* = 10.60 Hz, 1H, NH), 3.33 (t, *J* = 11.55 Hz, 1H, NH), 2.82 (tt, *J* = 6.44 Hz, 29.45 Hz, 2H, CH₂CF₂), 1.44 (d, *J=* 7.10 Hz, 3H, NHCHCH₃), 1.36 (d, *J=* 7.10 Hz, 3H, NHCHCH₃). ¹³C NMR (125 MHz, CDCl₃): 176.6, 135.0, 134.6, 128.7, 128.6, 128.5, 128.4, 128.3, 128.2, 127.4, 121.5, 67.3, 48.7, 37.9, 21.3. ³¹P NMR (202 MHz, CDCl₃): 12.76 (t, *J* = 95.62 Hz). ¹⁹F NMR (470 MHz, CDCl₃): -109.99 (d, *J=* 96.26 Hz), -110.56 (d, *J=* 39.79 Hz), -110.76 (d, *J* = 39.38 Hz), -111.32 (d, *J* = 94.75 Hz).

**Dimethyl 2,2'-((but-3-en-1-ylphosphoryl)bis(azanediyl))(25,2'S)-dipropionate (13a).** Yield: 424 mg (44%). ¹H NMR (500 MHz, CDCl₃): 5.86 (m, 1H, CH₂=CH-), 5.07 (m, 2H, CH₂=CH-), 4.03 (m, 2H, 2×CHNH), 3.74 (d, *J =* 2.3 Hz, 6H, OCH₃), 3.03, 2.93 (m, 2×1H, 2×NH), 2.36 (m, 2H, PCH₂CH₂), 1.81 (m, 2H, PCH₂CH₂), 1.38 (d, *J* = 7.2 Hz, 6H, 2×NHCHCH₃). ¹³C NMR (125 MHz, CDCl₃): 174.5, 156.25, 137.55, 115.47, 52.36, 48.81, 48.42, 28.94, 28.04, 26.86, 21.56, 18.99. ³¹P NMR (202 MHz, CDCl₃): 28.78 (s).

**Diisopropyl 2,2'-((but-3-en-1-ylphosphoryl)bis(azanediyl))(25,2'S)-dipropionate (13b).** Yield: 300 mg (26.5%). ¹H NMR (500 MHz, CDCl₃): 5.86 (m, 1H, CH₂=CH-), 5.03 (m, 4H, CH₂=CH-, 2×CH(CH₃)₂), 3.96 (m, 2H, 2×CHNH), 3.07, 2.98 (m, 2×1H, 2×NH), 2.36 (m, 2H, PCH₂CH₂), 1.80 (m, 2H, PCH₂CH₂), 1.37 (m, 6H, 2×NHCHCH₃), 1.25 (m, 12H, OCH(CH₃)₂). ¹³C NMR (125 MHz, CDCl₃): 174.27, 137.49, 115.36, 68.95, 49.00, 48.63, 29.05, 28.15, 26.88, 21.69, 19. 16. ³¹P NMR (202 MHz, CDCl₃): 28.59 (s).

**Di-tert-butyl 2,2'-((but-3-en-1-ylphosphoryl)bis(azanediyl))(25,2'S)-dipropionate (13c).** Yield: 284 mg (23%). ¹H NMR (500 MHz, CDCl3): 5.84 (m, 1H, CH₂=CH-), 5.06 (m, 2H, CH₂=CH-), 3.90 (m, 2H, 2×CHNH), 3.03, 2.91 (m, 2×1H, 2×NH), 2.35 (m, 2H, PCH₂CH₂), 1.78 (m, 2H, PCH₂CH₂), 1.46 (m, 18H, 2×OC(CH₃)₃), 1.36 (m, 6H, 2×NHCHCH₃). ¹³C NMR (125 MHz, CDCl₃): 173.8, 137.56, 115.24, 81.63, 49.26, 29.11, 28.22, 27.97, 26.89, 21.76. ³¹P NMR (202 MHz, CDCl₃): 28.53 (s).

**Dibenzyl 2,2'-((but-3-en-1-ylphosphoryl)bis(azanediyl))(25,2'S)-dipropionate (13d).** Yield: 400 mg (28%). ¹H NMR (500 MHz, CDCl₃): 7.35 (m, 10H, Ph), 5.79 (m, 1H, CH₂=CH-), 5.15 (m, 4H, 2×OCH₂C₆H₅), 5.01 (m, 2H, CH₂=CH-), 4.05 (m, 2H, 2×CHNH), 3.00, 2.93 (m, 2×1H, 2×NH), 2.31 (m, 2H, PCH₂CH₂), 1.76 (m, 2H, PCH₂CH₂), 1.42 (m, 6H, 2×NHCHCH₃). ¹³C NMR (125 MHz, CDCl₃): 174.53, 137.54, 128.64, 128.45, 128.24, 115.44, 67.06, 48.76, 46.78, 28.98, 28.08, 26.84, 21.41, 8.63. ³¹P NMR (202 MHz, CDCl₃): 28.71 (s).

**General procedure for synthesizing 9a-d and 14a-d.** Compounds 8a-d and 13a-d (1 eq., 0.335 mmol) were dissolved in 10 mL DCM under nitrogen inert atmosphere. 2-methyl-2-propen-1-ol (0.056 mL, 2 eq., 0.67 mmol), 1,4-benzoquinone (3.6 mg, 10% mol, 0.0335 mmol) and Hoveyda-Grubbs catalyst 2^{nd} generation (5.22 mg added three times at 0, 3, 6 h (15.7 mg in total), 7.5% mol, 0.025 mmol,) were added. The mixture was refluxed at 45 °C for 18 h and then cooled to room temperature. Activated charcoal was added and stirred for 1 h to absorb inorganic Ru from the catalyst. After that, the mixture was filtered through Celite, concentrated under reduced pressure and purified by flash column chromatography (EtOAc: Hexane, gradient from 20:80 to 100:0). Geometric configuration confirmed by NOESY.

**Dimethyl 2,2'-((((E)-1,1-difluoro-5-hydroxy-4-methylpent-3-en-1-yl)phosphor-yl)bis(azanediyl))(2S,2'S)-dipropionate** (9a). Yield: 62 mg (38%). ¹H NMR (500 MHz, CDCl₃): 5.53 (m, 1H, CH₂CH=CCH₃CH₂OH), 4.11 (m, 2H, 2×NHCH), 4.04 (s, 2H, CH₂OH), 3.76 (s, 6H, 2×OCH₃), 3.62(m, 1H, NHCH), 3.42 (m, 1H, NHCH), 2.91 (m, 2H, CF₂CH₂), 1.72 (s, 3H, CH=CCH₃CH₂OH), 1.44 (t, *J* = 6.83 Hz, 6H, 2×NHCHCH₃). ¹³C NMR (125 MHz, CDCl₃): 176.58, 142.01, 113.34, 67.95, 52.69, 48.78, 32.56, 21.77, 21.41, 14.00. ³¹P NMR (202 MHz, CDCl₃): 13.31 (t, *J* = 96.83 Hz). ¹⁹F NMR (470 MHz, CDCl₃): -108.32 (d, *J* = 96.68 Hz), - 108.91 (d, *J* = 66.49 Hz), -109.11 (d, *J* = 66.42 Hz), -109.70 (d, *J* = 96.49 Hz). HRMS (ES+, m/z): calcd for (M + Na)⁺ C₁₄H₂₅F₂N₂O₆PNa, 409.1317; found, 409.1316. HPLC (reverse-phase) 0.5 mL/min MeOH/H₂O 70:30 in 12 min, λ = 210 nm, tRt = 5.82 min (100%).

**Diisopropyl 2,2'-((((E)-1,1-difluoro-5-hydroxy-4-methylpent-3-en-1-yl)phosphor-yl)bis-(azanediyl))(2S,2'S)-dipropionate (9b).** Yield: 174 mg (52%). ¹H NMR (500 MHz, CDCl₃): 5.54 (m, 1H, CH=C), 5.04 (m, 2H, 2 × OCHCH₃), 4.05 (m, 4H, CH₂OH, 2 × CHNH), 3.57, 3.32 (m, 2 × 1H, 2 × NH), 2.89 (m, 2H, POCF₂CH₂), 2.30 (s, 1H, OH), 1.73 (s, 3H, CH₃(CH₂OH)C=CH), 1.42 (t, *J =* 6.7 Hz, 6H, 2 × NHCHCH₃), 1.26 (m, 12H, OCH(CH₃)₂). ¹³C NMR (125 MHz, CDCl₃): 173.6, 142.2, 113.5, 69.5, 68.0, 49.0, 32.7, 22.0, 21.6, 21.4, 14.0. ³¹P NMR (202 MHz, CDCl₃): 13.18 (t, *J* = 96.56 Hz). ¹⁹F NMR (470 MHz, CDCl₃): - 108.85 (d, *J* = 11.95 Hz), -109.06 (d, *J* = 11.58 Hz). HRMS (ES+, m/z): calcd for (M + Na)⁺ C₁₈H₃₃F₂N₂O₆PNa, 465.1950; found, 465.1942. HPLC (reverse-phase) 0.5 mL/min MeOH/H₂O 70:30 in 12 min, λ = 210 nm, tRt = 5.82 min (100%).

**Di-tert-butyl 2,2'-((((E)-1,1-difluoro-5-hydroxy-4-methylpent-3-en-1-yl)phosphoryl)bis(azanediyl))(2S,2'S)-dipropionate (9c).** Yield: 81 mg (67%). ¹H NMR (500 MHz, CDCl₃): 5.54 (m, 1H, CH=C), 4.05 (s, 2H, CH₂OH), 3.97 (m, 2H, 2 × CHNH), 3.53, 3.30 (m, 2 × 1H, 2 × NH), 2.89 (m, 2H, POCF₂CH₂), 2.30 (s, 1H, OH), 1.73 (s, 3H, CH₃(CH₂OH)C=CH), 1.47 (d, *J=* 4.0 Hz, 18H, 2 × OC(CH₃)₃), 1.40 (m, 6H, 2 × NHCHCH₃). ¹³C NMR (125 MHz, CDCl₃): 173.35, 142.15, 113.62, 82.38, 68.09, 49.47, 32.67, 27.92, 21.85, 14.04. ³¹P NMR (202 MHz, CDCl₃): 13.19 (t, *J =* 96.31 Hz). ¹⁹F NMR (470 MHz, CDCl₃): -108.85 (d, *J* = 7.72 Hz), -109.06 (d, *J* = 8.39 Hz). HRMS (ES+, m/z): calcd for (M + Na)⁺ C₂₀H₃₇F₂N₂O₆PNa, 493.2260; found, 493.2255. HPLC (reverse-phase) 0.5 mL/min MeOH/H₂O 70:30 in 12 min, λ = 210 nm, tRt = 5.81 min (100%).

**Dibenzyl 2,2'-((((E)-1,1-difluoro-5-hydroxy-4-methylpent-3-en-1-yl)phosphoryl)-bis(azanediyl))(2S,2'S)-dipropionate (9d).** Yield: 76 mg (57%). ¹H NMR (500 MHz, CDCl₃): 7.34 (m, 10H, Ph), 5.51 (m, 1H, CH=C), 5.15 (m, 4H, 2 × OCH₂C₆H₅), 4.13 (m, 2H, 2 × CHNH), 4.03 (s, 2H, CH₂OH), 3.55, 3.35 (m, 2 × 1H, 2 × NH), 2.87 (m, 2H, POCF₂CH₂), 2.16 (s, 1H, OH), 1.70 (s, 3H, CH₃(CH₂OH)C=CH), 1.44, 1.37 (2m, 2 ×3 H, 2 × NHCHCH₃). ¹³C NMR (125 MHz, CDCl₃): 173.76, 142.07, 135.15, 128.66, 128.54, 128.27, 113.38, 67.70, 64.38, 60.41, 48.83, 32.56, 31.34, 14.02. ³¹P NMR (202 MHz, CDCl₃): 13.14 (t, *J* = 96.70 Hz). ¹⁹F NMR (470 MHz, CDCl₃): -108.36 (d, *J=* 96.43 Hz), -108.94 (d, *J=* 59.26 Hz), -109.15 (d, *J* = 58.87 Hz), -109.72 (d, *J* = 96.68 Hz). HRMS (ES+, m/z): calcd for (M + Na)⁺ C₂₆H₃₃F₂N₂O₆PNa, 561.1940; found, 561.1942. HPLC (reverse-phase) 0.5 mL/min MeOH/H₂O 70:30 in 12 min, λ = 210 nm, tRt = 5.53 min (100%).

**Dimethyl 2,2'-((((E)-5-hydroxy-4-methylpent-3-en-1-yl)phosphoryl)bis(az-anediyl))-(2S,-2'S)-dipropionate (14a).** Yield: 66 mg (63%). ¹H NMR (500 MHz, CDCl₃): 5.45 (m, 1H, CH=C), 4.05 (m, 2H, 2 × CHNH), 4.01 (s, 2H, CH₂OH), 3.74, 3.73 (2×s, 2 × 3H, 2 × OCH₃), 3.02 (m, 2H, 2 × NH), 2.39 (m, 2H, PCH₂CH₂), 2.01 (s, 1H, OH), 1.79 (m, 2H, PCH₂CH₂), 1.65 (s, 3H, CH₃(CH₂OH)C=CH), 1.40 (m, 6H, 2 × NHCHCH₃). ¹³C NMR (125 MHz, CDCl₃): 175.33, 137.04, 124.07, 68.41, 52.44, 48.62, 29.64, 28.75, 21.62, 21.06, 13.80. ³¹P NMR (202 MHz, CDCl₃): 29.05 (s). HRMS (ES+, m/z): calcd for (M + Na)⁺ C₁₄H₂₇N₂O₆PNa, 373.1498; found, 373.1504. HPLC (reverse-phase) 0.5 mL/min MeOH/H₂O 70:30 in 12 min, λ = 210 nm, tRt = 5.80 min (98%).

**Diisopropyl 2,2'-((((E)-5-hydroxy-4-methylpent-3-en-1-yl)phosphoryl)bis(az-anediyl))-(2S,2'S)-dipropionate (14b).** Yield: 40 mg (33%). ¹H NMR (500 MHz, CDCl₃): 5.46 (m, 1H, CH=C), 5.01 (m, 2H, 2 × OCH(CH₃)₂), 4.00 (s, 2H, CH₂OH), 3.97 (m, 2H, 2 × CHNH), 3.05 (m, 2H, 2 × NH), 2.38 (m, 2H, PCH₂CH₂), 1.80 (m, 2H, PCH₂CH₂), 1.70 (s, 3H, CH₃(CH₂OH)C=CH), 1.38 (m, 6H, 2 × NHCHCH₃), 1.25 (m, 12H, 2 × OCH(CH₃)₂). ¹³C NMR (125 MHz, CDCl₃): 174.39, 137.13, 124.14, 69.09, 68.44, 48.90, 29.78, 28.88, 21.89, 21.67, 21.12, 13.81. ³¹P NMR (202 MHz, CDCl₃): 28.95 (s). HRMS (ES+, m/z): calcd for (M + Na)⁺ C₁₈H₃₅N₂O₆PNa, 429.2143; found, 429.2130. HPLC (reverse-phase) 0.5 mL/min MeOH/H₂O 70:30 in 12 min, λ = 210 nm, tRt = 5.81 min (100%).

**Di-tert-butyl 2,2'-((((E)-5-hydroxy-4-methylpent-3-en-1-yl)phosphoryl)bis(az-ane-diyl))-(2S,2'S)-dipropionate (14c).** Yield: 43 mg (33%). ¹H NMR (500 MHz, CDCl₃): 5.45 (m, 1H, CH=C), 4.01 (s, 2H, CH₂OH), 3.92 (m, 2H, 2 × CHNH), 3.02 (m, 2H, 2 × NH), 2.38 (m, 2H, PCH₂CH₂), 1.77 (m, 2H, PCH₂CH₂), 1.71 (s, 3H, CH₃(CH₂OH)C=CH), 1.46 (d, *J* = 4.6 Hz, 18H, 2 × OC(CH₃)₃), 1.36 (dd, *J* = 13.7, 7.1 Hz, 2×NHCHCH₃). ¹³C NMR (125 MHz, CDCl₃): 174.12, 137.18, 124.27, 81.82, 68.52, 49.23, 29.82, 28.94, 27.98, 22.08, 21.16, 13.82. ³¹PNMR (202 MHz, CDCl₃): 28.90 (s). HRMS (ES+, m/z): calcd for (M + Na)⁺ C₂₀H₃₉N₂O₆PNa, 457.2453; found, 457.2443. HPLC (reverse-phase) 0.5 mL/min MeOH/H₂O 70:30 in 12 min, λ = 210 nm, tRt = 5.54 min (98%).

**Dibenzyl 2,2'-((((E)-5-hydroxy-4-methylpent-3-en-1-yl)phosphoryl)bis(azane-diyl))-(2S, 2'S)-dipropionate (14d).** Yield: 128 mg (59%). ¹H NMR (500 MHz, CDCl₃): 7.34 (m, 10H, Ph), 5.40 (m, 1H, CH=C), 5.14 (m, 4H, 2 × OCH₂C₆H₅), 4.06 (m, 2H, 2 × CHNH), 3.99 (s, 2H, CH₂OH), 3.02 (m, 2H, 2 × NH), 2.34 (m, 2H, PCH₂CH₂), 1.74 (m, 2H, PCH₂CH₂), 1.67 (s, 3H, CH₃(CH₂OH)C=CH), 1.41, 1.32 (2d, 2 × 3H, *J* = 7.0 Hz, 2 × NHCHCH₃). ¹³C NMR (125 MHz, CDCl₃): 174.59, 136.99, 135.29, 128.64, 128.48, 128.25, 124.11, 68.41, 67.77, 48.76, 29.66, 28.75, 21.63, 21.05, 13.79. ³¹P NMR (202 MHz, CDCl₃): 29.10 (s). HRMS (ES+, m/z): calcd for (M + Na)⁺ C₂₆H₃₅N₂O₆PNa, 525.2127; found, 525.2130. HPLC (reverse-phase) 0.5 mL/min MeOH/H₂O 70:30 in 12 min, λ = 210 nm, tRt = 5.82 min (100%).

To ensure the samples used were not compromised upon dissolution and storage, the samples were again analysed by mass spectrometry and HPLC, confirming they were structurally correct and pure.

### Example 2: Stability studies of HMBP difluoromethylphosphonate 9b, a ProPAgen according to the invention

In order to demonstrate the stability of the phosphonodiamidate ProPAgens according to the present invention in human serum, a representative serum stability study was conducted with difluoromethylphosphonate 9b, which has an iPr ester like the two FAD-approved phosphoramidate prodrugs sofosbuvir and tenofovir alafenmide.

This experiment was carried out as previously reported in Mehellou et al. 2020 and Slusarczyk, M.; Ferrari, V.; Serpi, M.; Gönczy, B.; Balzarini, J.; McGuigan, C; Symmetrical Diamidates as a Class of Phosphate Prodrugs to Deliver the 5'-Monophosphate Forms of Anticancer Nucleoside Analogues; ChemMedChem 2018, 13, 2305-2316 (hereinafter "McGuigan et al. 2018"), as well as Slusarczyk, M.; Lopez, M. H.; Balzarini, J.; Mason, M.; Jiang, W. G.; Blagden, S.; Thompson, E.; Ghazaly, E.; McGuigan, C. Application of ProTide technology to gemcitabine: a successful approach to overcome the key cancer resistance mechanisms leads to a new agent (NUC-1031) in clinical development. J. Med. Chem. 2014, 57, 1531-1542 (hereinafter "McGuigan et al. 2014").

Briefly, 5.0 mg of the phosphonodiamidate ProPAgen 9b was dissolved in a mixture of 0.05 mL of DMSO and 0.15 mL D₂O. After recording the first ³¹P NMR data, 0.3 mL human serum (Merck Life Sciences) was added and monitored by NMR. The experiment was run on NMR ³¹P mode and scanned every half an hour for 7.5 h. The incubation temperature was 37°C. Recorded data were processed and analysed with Bruker Topspin 2.1 software.

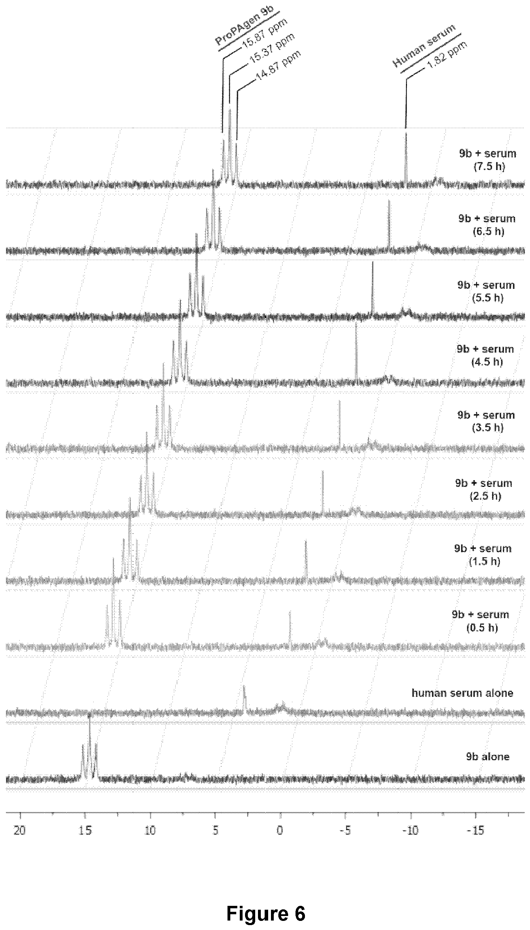

ProPAgen 9b was incubated with human serum at 37 °C for 7.5 h and monitored by ³¹P NMR spectroscopy. As shown in Figure 6, the ³¹P NMR spectra of ProPAgen 9b showed three phosphorous peaks (*δ*_{P} = 14.87, 15.37 and 15.87 ppm) due to the coupling with the fluorine atoms, a typical and expected pattern of the ³¹P-NMR of these prodrugs. Notably, the human serum also showed a ³¹P-NMR peak at *δ*_{P} = 1.82. Following the incubation of the phosphonodiamidate ProPAgen 9b with human serum addition of human serum and monitoring of the sample by ³¹P-NMR, these original 9b ³¹P-NMR peaks remained present through the 7.5 h of the study, and no new ³¹P -NMR peaks were observed. These data indicate excellent human serum stability of 9b (t_{1/2} > 7.5 h).

This stability profile is in line with the aryloxy diester phosphoramidate prodrugs of these monophosphonates that are reported Mehellou et al. 2020.

### Example 3: Activation of human Yγ9/Vδ2⁺ T cells

**General:** Healthy donor peripheral blood mononuclear cells (PBMCs) were harvested as described in Morita et al. 2007. In particular, blood was obtained in the presence of a mixture of heparin and ethylene diamino tetra acetic acid (EDTA) as anticoagulants (2 U/ml heparin, 1.5 mM EDTA) from consented healthy donors (approved by the NRES Committee West Midlands-Solihull Ethical Board; REC reference 14/WM/1254). Blood was then layered on a density gradient medium, lymphoprep (Stem Cell Technologies) and Peripheral blood mononuclear cells (PBMCs) were purified by gradient centrifugation. The cells were washed 2 times with Phosphate Buffered Saline (PBS), then resuspended in RPMI-1640 media supplemented with 2 mM L-glutamine, 25 mM HEPES, 1% sodium pyruvate, 50 µg/ml penicillin/streptomycin (Invitrogen) and 10% foetal calf serum.

To assess the activation of Vy9/V62 T cells, PBMCs were seeded into U-bottom tissue culture-treated 96-well plates at a cell density of 500,000 cells per well. The cells were incubated with medium-only (control), in the presence of Zoledronate and HMBPP at 10pM to 100µM, and with HMBP ProPAgens of the present invention, namely ProPAgens 9a-d and 14 a-d, at concentrations of initially 10pM to 100µM, and then at 1aM to 100µM for compounds 9d and 14d in a separate experiment. The cells were incubated at 37°C/5%CO₂ overnight, and stained by flow cytometry for the following markers: viability (Zombie Aqua 1:400), CD3 (BV421 1:100), CD8 (BV650 1:200), Vγ9 (PEcy5 1:400), V62 (APC 1:200), CD69 (PE 1:25), CD25 (FITC 1:100). The samples were acquired via LSRFortessa X20 (BD Biosciences), and the data obtained were analysed using FlowJo v10 and GraphPad Prism v9 software.

Peripheral blood γδ T-cells lack appreciable levels of surface CD69 or CD25 under steady state conditions, but T-cell receptor (TCR) stimulation upregulates both T-cell activation markers within 72 hours. PAg responsive Vy9/V62 T-cells were then distinguished by TCR Yγ9 and V62 expression and assessed for the upregulation CD69 and CD25.

### Example 3-1: Activation of human Vγ9/Vδ2⁺ T cells by ProPAgens 9a-d and 14 a-d

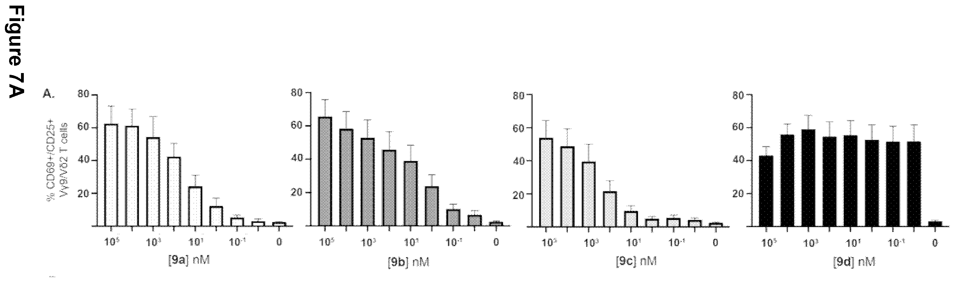

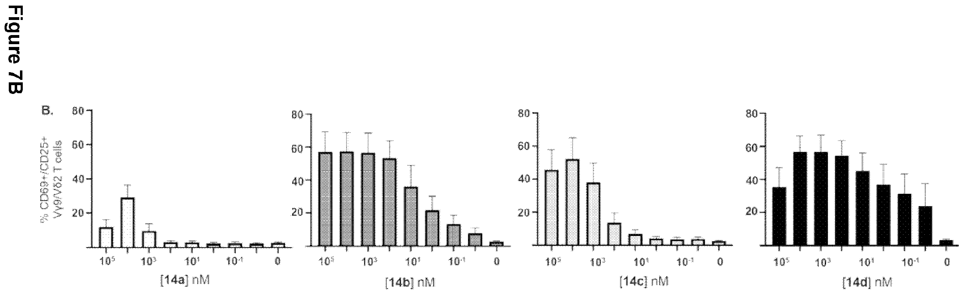

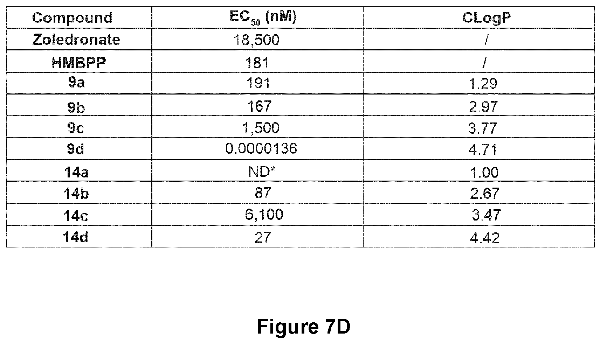

To demonstrate activation of Vy9/V62 T-cells by ProPAgens 9a-d and 14a-d and subsequent *in vitro* lysis of cancer cells, peripheral blood mononuclear cells (PBMCs) containing Vy9/V62 T-cells derived from healthy donors were incubated with increasing concentrations of phosphonodiamidate HMBP ProPAgens 9a-d and 14a-d (up to 100 µM) as described above, see Figures 7A, 7B and 7D.

As for the activation of Vy9/V62 T cells by the phosphonodiamidate ProPAgens (9a-d and 14a-d), these were initially tested using a concentration range from 0.1 nM to 100 µM (Figures 7A and 7B). The results showed that these phosphonodiamidate ProPAgens exhibited varying levels of activation that range from super potent activation EC₅₀ = 0.0000136 nM to 6.1 µM. In both the fluorinated (9a-d) and non-fluorinated (14a-d) series, the phosphonodiamidate ProPAgens bearing a tert-butyl ester (9c and 14c) exhibited the least potent activation of Vy9/V62 T cells, EC₅₀ = 1.5 and 6.1 µM, respectively (Figures 7A, 7B and 7D). This was followed by the methyl ester phosphonodiamidate prodrugs where 9a exhibited good potency, EC₅₀ = 191 nM, though we were unable to obtain an accurate potency level of 14a.

Across the two series of the phosphonodiamidate ProPAgens 9a-d and 14a-d, those bearing an isopropyl or benzyl esters exhibited the most potent activation of Yγ9/Vδ2 T cells *in vitro.* Phosphonodiamidate ProPAgens 9b and 14b showed good activation of Yγ9/Vδ2 T cells, EC₅₀ = 167 and 87 nM, respectively. However, the phosphonodiamidate ProPAgens, 9d and 14d exhibited the most potent activation of Yγ9/Vδ2 T cells and ProPAgen 9d was the most potent in terms of Yγ9/Vδ2 T cell activation across the eight ProPAgens studied in this work, EC₅₀ = 13.6 fM (Figures 7A, 7B and 7D).

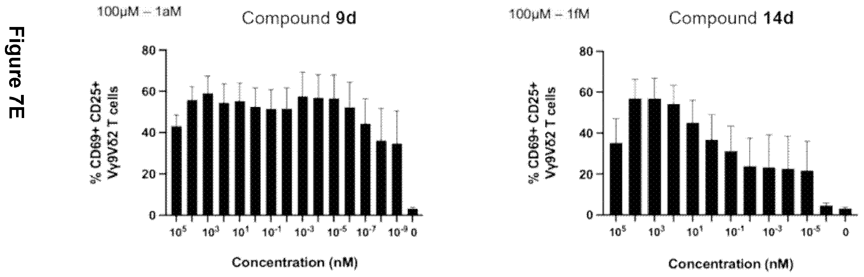

In order to determine the accurate potencies (EC₅₀) for the two benzyl phosphonodiamidate prodrugs 9d and 14d, we performed the activation assay with a concentration range of 10 aM to 100 µM (Figure 7E).

Notably, the extremely high Vy9/V62 T cell activation potency of the phosphonodiamidate ProPAgen 9d is comparable to its corresponding aryloxy diester phosphoramidate derivative, EC₅₀ = 9.15 fM. Notably, at the highest concentration studied (100 µM), the activation of Vy9/V62 T cells by the phosphonodiamidate ProPAgens 9d and 14d was less than that achieved with 10 µM (Figures 4A and 4B). This could be explained by negative feedback mechanisms induced by antigen overstimulation that lead to downregulation of the TCR, subsequently resulting in lower expression of the CD25 activation marker. This was also observed with the aryloxy diester phosphoramidate prodrug of HMBP methylene and difluoromethylene monophosphonates.

### Example 3-2 (Comparative): Activation of Vγ9/Vδ2⁺ T cells by HMBPP and zoledronate

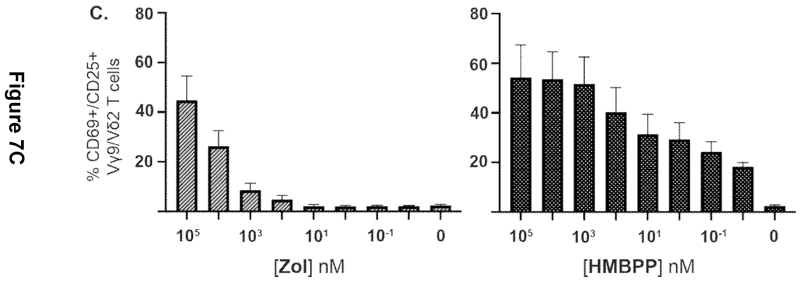

In positive control experiments, HMBPP and zoledronate (Zol) were used in the activation assays as described above. HMBPP and zoledronate (Zol) exhibited significant activation of Vy9/Vδ2 T cells, EC₅₀ = 181 nM and 18.5 µM, respectively (Figures 7C and 7D). Notably, these potencies by HMBPP and zoledronate are lower in terms of potency compared to previously reported potencies for these compounds (EC₅₀ = 60-500 pM for HMBPP and 0.003-0.5 µM for zoledronate), see Hsiao, C. H.; Lin, X.; Barney, R. J.; Shippy, R. R.; Li, J.; Vinogradova, O.; Wiemer, D. F.; Wiemer, A. J. Synthesis of a phosphoantigen prodrug that potently activates Vgamma9Vdelta2 T-lymphocytes. Chem. Biol. 2014, 21 (8), 945-954 and Reichenberg, A.; Hintz, M.; Kletschek, Y.; Kuhl, T.; Haug, C.; Engel, R.; Moll, J.; Ostrovsky, D. N.; Jomaa, H.; Eberl, M. Replacing the pyrophosphate group of HMB-PP by a diphosphonate function abrogates Its potential to activate human gammadelta T cells but does not lead to competitive antagonism, Bioorg. Med. Chem. Lett. 2003, 13, 1257-1260.

Such differences between previously reported HMBPP and zoledronate potencies compared to those observed in this study are most likely due to modest inter-individual variations in the activity of Vy9/Vδ2 T cells.

As is evident upon comparison of Figures 7A-7E, the T-cell activation potency of HMBPP and zoledronate is inferior to that activation potency of ProPAgens according to the present invention, except for compounds 9a, 9c and 14c.

### Example 3-3: Lack of Activation of human CD8+ Tcells by ProPAgens 4a-d and 9a-d

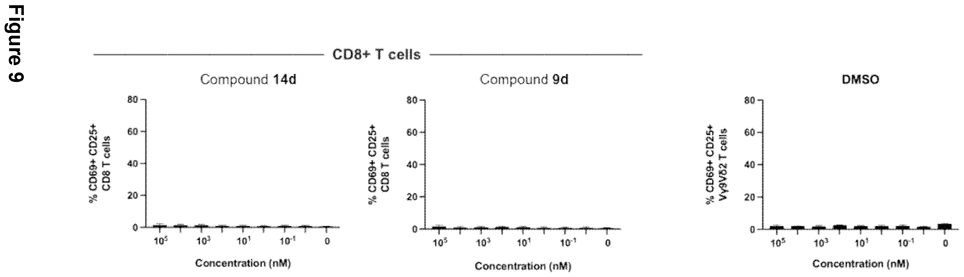

To demonstrate that the ProPAgens of the present invention are Vy9/V62 T cell - specific activators, PBMCs containing CD8⁺ αβ T-cells derived from donors were incubated with increasing concentrations of ProPAgens 9d and 14d (Figure 9).

Like peripheral blood γδ T-cells, peripheral blood CD8⁺ T-cells lack appreciable levels of surface CD69 or CD25 under steady state conditions, but upon T-cell receptor (TCR) stimulation upregulate both T-cell activation markers within 72 hours. PAg responsive CD8 T-cells were then distinguished by TCR CD8 expression and assessed for the upregulation CD69 and CD25.

As shown in Figure 9, HMBP phosphonate PropAgens 9d and 14d, as representatives of these classes of prodrugs according to the present invention, did not exhibit activation of CD8⁺ T cells, even upon incubation at a concentration of 1 µM, i.e. at concentrations approx. 100,000 times greater than the concentration for which Yγ9/Vδ2 T-cell activation EC₅₀ values were determined for both ProPAgens 9d and 14d.

### Example 4: Cytotoxicity assay showing potent lysis of T24 bladder cancer cells by Yγ9/Vδ2 T-cells is mediated and enhanced by ProPAgens 9d and 14d

As a further proof of principle, and to demonstrate that the superior Vγ9/Vδ2⁺ T cell activation efficacy of ProPAgens of the present invention shown above does translate into a beneficial therapeutic effect, the specific lysis of cancer cells by *in vitro* expanded Yγ9/Vδ2 T-cells was studied, see Figure 8:
To assess the level of killing of drug-treated and untreated tumour cells, a Europium-based cytotoxicity assay was performed (DELFIA, Perkin Elmer) as described in Fisher et al. 2014.

Human T24 urinary bladder carcinoma cell lines were cultured in PBS for 2 h at 37°C/5% CO₂ without ProPAgens (i.e. untreated), containing 10 µM zoledronate (i.e. the clinically used small molecule drug) or the indicated HMBP phosphonodiamidate ProPAgens of the present invention, i.e. ProPAgen 9d at a concentration of 10nM, and ProPAgen 14d at a concentration of 10nM. A positive control for cell death (target cells incubated with 10% v/v DELFIA lysis buffer) and media-only treated control (no drug) were also included. The cells were then washed 3 times at 600 x g for 5 min in PBS to remove any excess drug and incubated in PBS with BATDA labelling agent (used at 1µl per ml) for 20 min at 37°C as before.

In the meantime, *ex vivo* expanded Vy9/V62 T cells, i.e. Vy9/V62 T cells that have been expanded with 5µM Zoledronate and 100U/ml IL2 over a period of 14 days, were thawed, counted and resuspended to a cell concentration of 4 x 106 cells/ml. After the BATDA labelling, T24 cells were washed 3 times in medium at 4°C and resuspended to a concentration of 5 × 104 cells/ml.

100 µl of T24 cells were then seeded into U-bottom tissue culture 96-well plates and co-cultured with 100 µl/well Vy9/V62 T cells (i.e. at an effector:target ratio of 80:1) as previously described in Fisher et al. 2014. In particular, drug-treated and untreated T24 cells were also seeded alone without Vy9/V62 effectors and 100 µl media was added per well instead. To the positive killing control, 10% v/v lysis buffer was added to drug-untreated T24 cells. The plate was centrifuged at 200 x g for 2 min to bring cells into contact in the co-cultures. The plate with all samples was incubated at 37 °C/5% CO₂ for 1 h. Following this incubation, the plate was centrifuged again at 600 x g for 2 min and 25 µl of the supernatant was transferred into a flat-bottom 96-well optical plate, to which Europium solution was added at 200µl per well.

The level of killing of T24 cells was then measured via time-resolved fluorescence using a PHERAstar microplate reader (BMG Labtech). Specific lysis (% killing of T24 cells) was calculated as follows: [(experimental release - spontaneous release)/(maximal release - spontaneous release)] x 100. The data were processed and analyzed using Microsoft Excel and GraphPad Prism v9 software. Data is shown in Figure 8 as mean ± SE (n = 7). Statistical analysis was performed using one-way ANOVA and Tukey's multiple comparisons test on GraphPad Prism v9. **p < 0.0063. The data show that the sensitizing effects of 10 nM phosphonodiamidate ProPAgens 9d and 14d was evidently much more potent in comparison to 10 µM Zoledronate.

As shown in Figure 8, the sensitizing effects of 10 nM phosphonodiamidate ProPAgens 9d and 14d were evidently much more potent in comparison to 10 µM zoledronate.

### Example 5: Metabolic study using a carboxypeptidase Y assay

The *in vitro* metabolism of the phosphonodiamidate ProPAgens according to the present invention was investigated in a carboxypeptidase Y assay.

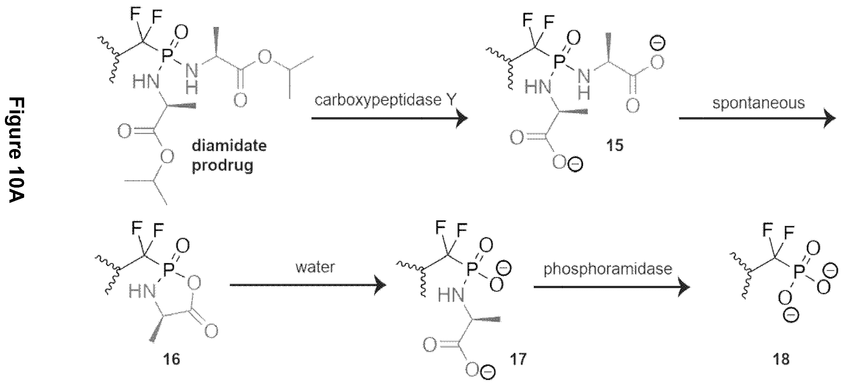

Based on metabolic studies reported by McGuigan et al. 2018 for phosphorodiamidate ProTides, and wishing not the be bound by theory, the postulated metabolism of ProPAgens according to the present invention is suggested to be initiated by an esterase, e.g., carboxypeptidase Y, which removes the ester motif from the amino acid esters and frees the carboxylate groups (metabolite 15 in Figure 10A). This is then followed by a spontaneous nucleophilic attack from one of the carboxylate groups onto the phosphorous center, which triggers leaving of the second amino acid and the generation of an unstable five membered ring (metabolite 16 in Figure 10A). The next metabolism step involves a nucleophilic attack from a water molecule onto the phosphorous or carbonyl group to generate phosphoramidate (metabolite 17 in Figure 10A). Finally, a phosphoramidase-type enzyme, e.g. Hint-1, cleaves off the P-N bond in 17, leading to the release of the unmasked monophosphonate species (metabolite 18 in Figure. 10A).

The carboxypeptidase Y assay was carried out as previously reported by Mehellou et al. 2020, McGuigan et al. 2018 and McGuigan et al. 2014. 5.0 mg of the phosphonodiamidate ProPAgen 9b was dissolved in 0.2 mL of acetone, and 0.4 mL of Trizma buffer (pH 7.4) was added followed by 0.5 mg carboxypeptidase Y in 0.2 ml Trizma buffer (pH 7.4). The phosphonodiamidate ProPAgen 9b was incubated with recombinant carboxypeptidase Y at 37 °C and monitored the reaction by ³¹P-NMR for 12 h. Recorded data were processed and analysed with Bruker Topspin 2.1 software.

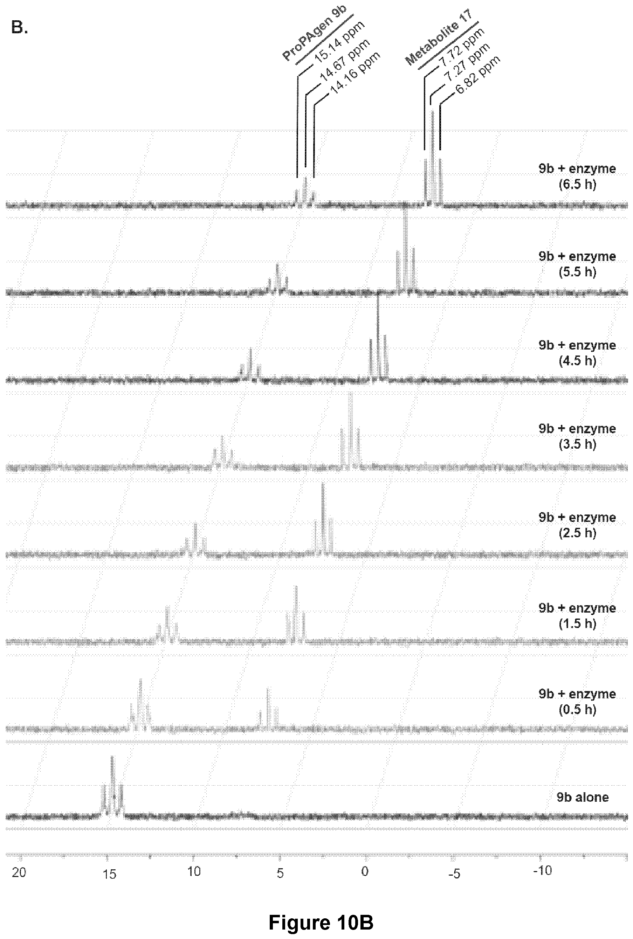

The results demonstrated that at t = 0 in the buffer of the reaction, ProPAgen 9b showed three ³¹P NMR peaks (*δ*P = 14.16, 14.67 and 15.14 ppm) as expected (see Figure 10B, 31P NMR spectrum recorded at 0 h incubation time). Upon addition of carboxypeptidase Y and within 0.5 h three new ³¹P NMR peaks (*δ*P = 6.82, 7.27 and 7.72 ppm) became prominent as the assay proceeded and within the 6.5 h of this study, these new peaks were the most prominent while those of 9b were the minor ³¹P NMR peaks (see Figure 10B, 31P NMR spectrum recorded at 6.5 h incubation time). The new ³¹P NMR shift of the new peaks that emerged correspond to that of metabolite 17 shown in Figure 10A, akin to what was observed for this metabolite (*δ*P = 6.50, 6.90 and 7.20 ppm) previously.

## Claims

1. A compound according to General Formula (I) including all tautomers thereof: 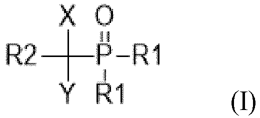 wherein R1 represents an amino acid ester radical according to General Formula (II): 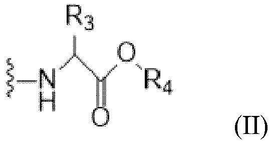 wherein R3 represents H, or a saturated or unsaturated and optionally substituted hydrocarbon chain;
R4 represents a saturated or unsaturated and optionally substituted hydrocarbon chain; and wherein both R1 are identical; and
R2 represents an optionally substituted C₂₋₂₀ alkyl, C₄₋₂₀ alkenyl or C₂₋₂₀ alcohol radical; and
each of X and Y independently represent H or halo;
or a salt thereof, and
wherein R1 is an amino acid ester radical according to General Formula (II) derived from alanine (R3 = -CH₃), leucine (R3 = -CH₂CH(CH₃)₂), isoleucine (R3 = - CH(CH₃)CH₂CH₃) or methionine (R3 = -CH₂CH₂SCH₃), most preferably R1 is an amino acid ester radical according to General Formula (II) derived from alanine (R3 = -CH₃); and
wherein R2 is a radical according to Formula (III) or Formula (IV), wherein R5 in Formula (IV) is selected from OH, OR6, SH, SR6, NH₂ or NHR6, and wherein R6 represents C₁₋₄ alkyl: 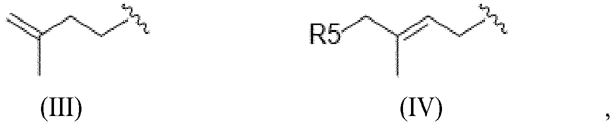
preferably, wherein R2 is a radical according to Formula (IV).

2. The compound according to claim 1, wherein R2 is a radical according to Formula (IV) and R5 is OH.

3. The compound according to any of the preceding claims, wherein at least one, and preferably both, of X and Y represent halogen.

4. The compound according to claim 3, wherein the one or both halo substituents is/are fluoro.

5. The compound according to any of the preceding claims, wherein R4 is an unsubstituted C₁₋₄ alkyl chain or an unsubstituted benzyl group, preferably, wherein R4 is selected from methyl, isopropyl, tert-butyl and benzyl, more preferably, wherein R4 is isopropyl or benzyl, most preferably wherein R4 is benzyl.

6. The compound according to any one of claims 1 to 5, wherein in R1 is an amino acid ester radical according to General Formula (II) derived from L-alanine, L-leucine, L-isoleucine or L-methionine, most preferably L-alanine.

7. The compound according to claim 6, wherein R4 is selected from methyl, isopropyl, tert-butyl and benzyl, preferably, wherein R4 is isopropyl or benzyl, most preferably, wherein R4 is benzyl.

8. The compound according to claim 1, selected from: 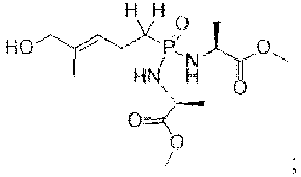 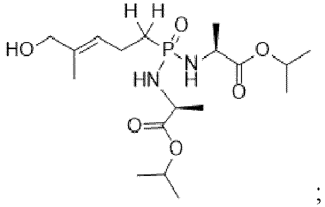 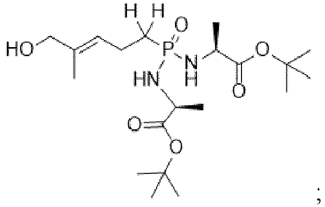 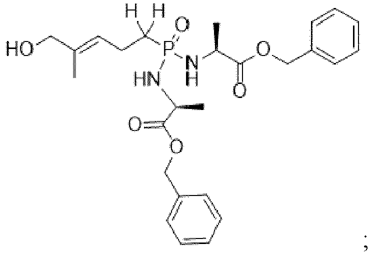 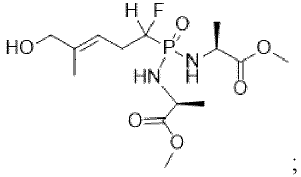 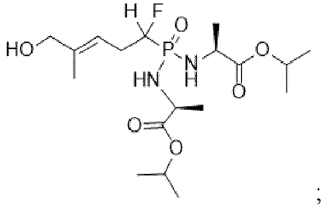 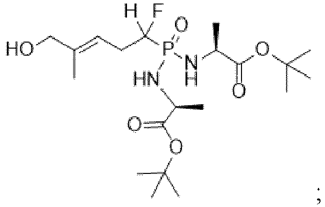 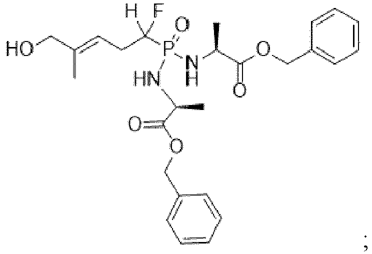 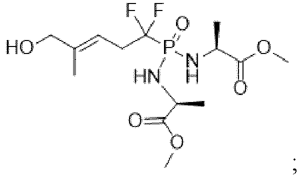 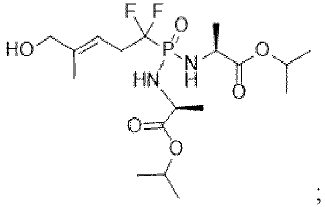 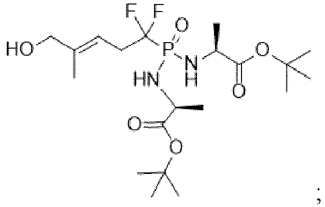 and 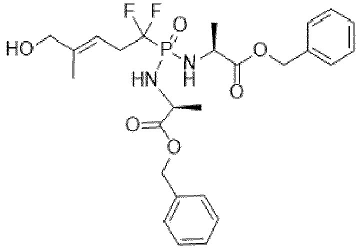 preferably the compound is selected from: 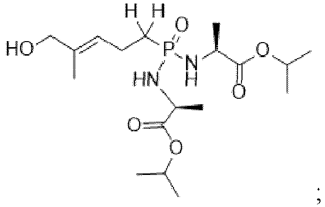 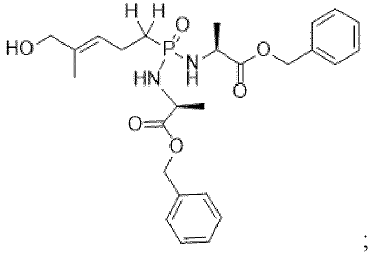 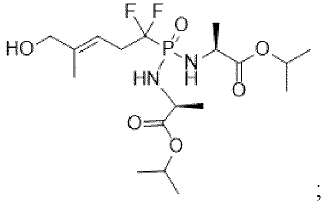 and 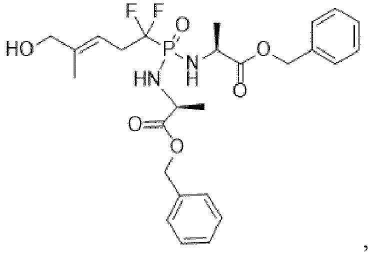 more preferably the compound is selected from: 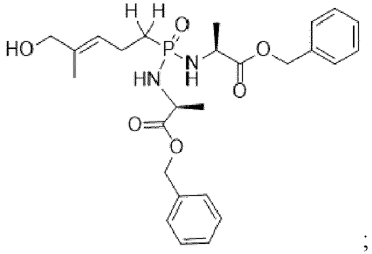 and 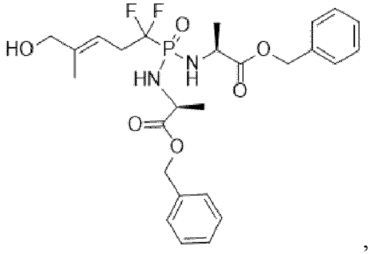 most preferably the compound is

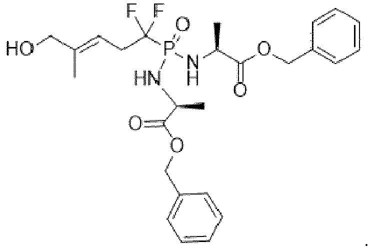

9. The compound according to claim 1, selected from: 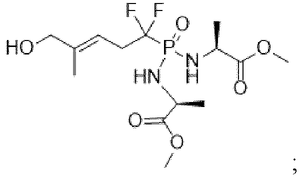 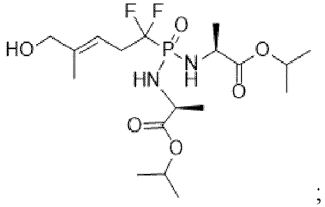 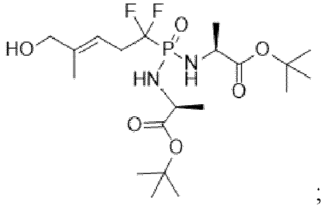 and 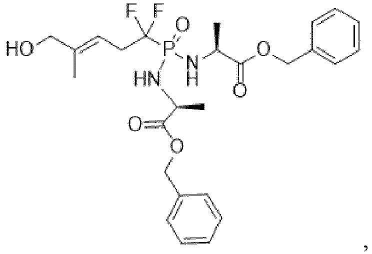 preferably the compound is

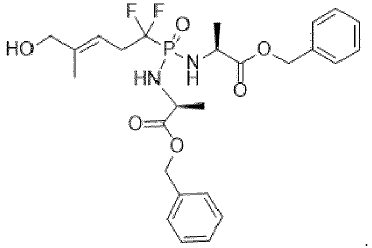

10. The compound according to claim 1, wherein the compound is

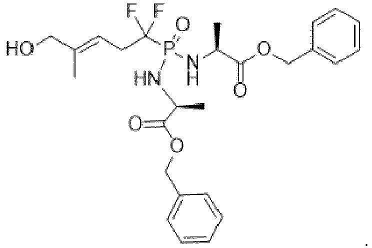

11. A pharmaceutical composition comprising a compound as defined in any one of claims 1 to 10, wherein the pharmaceutical composition preferably comprises a pharmaceutically acceptable excipient or carrier.

12. The compound according to any one of claims 1 to 10 or the pharmaceutical composition according to claim 11 for medical use.

13. The compound according to any one of claims 1 to 10 or the pharmaceutical composition according to claim 11 for use in immunotherapy.

14. The compound or pharmaceutical composition for use according to claim 13, wherein said immunotherapy is directed to the treatment of a proliferative disease, an infectious disease, an inflammatory disease and/or osteoporosis, and wherein said proliferative disease preferably is a cancer, and wherein the cancer is preferably selected from the group consisting of a hematologic cancer, bladder cancer, prostate cancer, lung cancer, neck cancer, skin cancer, breast cancer and mesothelioma.

15. The compound or pharmaceutical composition for use according to claim 13 or 14, wherein said immunotherapy
(a) comprises the administration of a therapeutically effective amount of the compound of any one of claims 1 to 10 to a subject in need thereof;
(b) comprises the administration of autologous or allogeneic T-cells, preferably γδ T cells, and more preferably Yγ9Vδ2 T-cells;
(c) further comprises the administration of a interleukin, preferably IL-2 and more preferably human IL-2; or
(d) comprises the activation of T-cells, preferably γδ T cells, even more preferably Yγ9Vδ2 T-cells.

16. An *ex vivo* method of preparing a population οf γδ T cells for therapeutic use, said method comprising:
(i) providing a population οf γδ T cells;
(ii) culturing the population οf γδ T cells in the presence of a compound according to any one of claims 1 to 10.

17. Population of yδ T cells obtained by the method according to claim 16, wherein said γδ T cells preferably are Yγ9/Vδ2 T cells.

18. A pharmaceutical composition comprising the population of yδ T cells according to claims 17.

19. The population of yδ T cells according to claim 17 or the pharmaceutical composition according to claim 18 for medical use.

20. The population οf γδ T cells according to claim 17 or the pharmaceutical composition according to claim 18 for use in immunotherapy.

21. The population of expanded γδ T cells or the pharmaceutical composition for use according to claim 20, wherein said immunotherapy is directed to the treatment of a proliferative disease, an infectious disease, an inflammatory disease and/or osteoporosis, wherein said proliferative disease preferably is a cancer, and wherein the cancer is preferably selected from the group consisting of a hematologic cancer, bladder cancer, prostate cancer, lung cancer, neck cancer, skin cancer, breast cancer and mesothelioma.

22. A process of synthesizing a compound as defined in any one of claims 1 to 10 comprising the steps of:
(i) providing a phosphonic acid alkyl ester;
(ii) converting the phosphonic acid alkyl ester into a phosphonic acid halogenide by removing the ester groups and subsequently subjecting to a halogenation reaction;
(iii) subjecting the phosphonic acid halogenide to an esterification reaction with an amino acid ester hydrohalogenide to obtain a amino acid ester derivative; and
(iv) subjecting the amino acid ester derivative of step (iii) to an olefin metathesis with prevention of alkene isomerization.
